# EUROPEAN PATENT SPECIFICATION

(11) **EP 2 989 574 B1**
(45) Date of publication and mention of the grant of the patent: **07.11.2018**
(21) Application number: 14723749.9
(22) Date of filing: 28.04.2014
(51) Int. Cl.: G06F 19/00, G06F 21/12, H04L 12/911, G16H 40/40

(54) **TREATMENT APPARATUS WITH MEDICAL FEATURE ACTIVATION**
BEHANDLUNGSVORRICHTUNG MIT AKTIVIERUNG VON MEDIZINISCHER FUNKTION
APPAREIL DE TRAITEMENT À ACTIVATION DE FONCTION MÉDICALE

(30) Priority: 26.04.2013 US 201361816564 P; 04.02.2014 US 201414172099
(43) Date of publication of application: 02.03.2016
(73) Proprietor: Roche Diabetes Care GmbH, 68305 Mannheim (DE); F. Hoffmann-La Roche AG, 4070 Basel (CH)
(72) Inventor: BIRTWHISTLE, Daniel, Zionsville, Indiana 46077 (US); CARLSGAARD, Eric, Zionsville, Indiana 46077 (US); FLIS, Michael, Carmel, Indiana 46033 (US); FRIEDMAN, Kevin, Atlanta, Georgia 30319 (US); JENSEN, Hans P., Fishers, Indiana 46037 (US); MARKISOHN, David B., Indianapolis, Indiana 46256 (US); REINKE, Robert E., Indianapolis, Indiana 46236 (US)
(74) Representative: Richardt Patentanwälte PartG mbB
(86) International application number: PCT/EP2014/058607
(87) International publication number: WO 2014/174118

(56) References cited:
- WO-A1-2009/071188
- US-A1- 2012 096 451
- US-A1- 2012 232 520

## Description

### Field of the invention

The present disclosure relates to a mobile phone application for diabetes care that allows persons with diabetes to activate and use a medical feature of the application using data received from a healthcare professional.

### Background and related art

Persons with diabetes have difficulty regulating blood glucose levels in their bodies. As a consequence, many of these persons carry specialized electronic meters, called blood glucose meters, which allow them to periodically measure their glucose levels and take appropriate action such as administering insulin using an insulin pump. These persons may also carry with them a portable communication device, such as a mobile phone, a personal digital assistant, a tablet, or a similar mobile device. People often rely on their portable communication devices as primary means for planning, scheduling, and communicating with others. As a result, most portable communication devices are equipped with sophisticated software which provides user-friendly means for viewing and inputting data.

Persons with diabetes may wish to view and analyze results of a blood glucose measurement obtained from their glucose meter on their portable communication device. Additionally, these persons may wish to transmit the results to a healthcare professional using the portable communication device. Further, these persons may wish to receive bolus advice instruction from the healthcare professional on the portable communication device in order to manage administration of insulin. Accordingly, it is desirable to develop a diabetes management application that runs on a portable communication device and that allows persons with diabetes to transmit blood glucose measures to a healthcare professional and receive bolus advice instruction from the healthcare professional.
This section provides background information related to the present disclosure which is not necessarily prior art.

Patent application US 20121232520 A1 (SLOAN MARK K [US] ET AL) (2012-09-13) describes methods, systems and devices for detecting an analyte sample, determining an analyte concentration associated with the detected analyte sample, and storing the determined analyte concentration and a time associated with the determined analyte concentration. The application further describes methods, systems and devices for retrieving two or more stored analyte concentrations and determining an adjusted dose level based at least in part on a current dose level and data associated with the two or more retrieved analyte concentrations. For example, adjustments to dosage levels of long acting insulin may be provided to assist in the management of diabetes and related conditions.
International patent application WO 2009/071188 A1 (2009-06-11) describes a system and method for selectively configuring features and functions of a consolidated software application for particular end users.

### Summary

This application claims the benefit of U.S. Provisional Application No. 61/816,564 filed April 26, 2013.

In one aspect the invention relates to a treatment apparatus comprising an apparatus for administering a drug to a mammal, a glucose-meter and a mobile device and relates to a method for activating a medical feature of an diabetes management application executed on a mobile device and on a glucose meter as specified in the independent claims. Embodiments of the invention are given in the dependent claims.

The mobile device is connected to a server via a network. The mobile device hosts an application program that comprises one or more medical features, i.e., modular pieces of program logic. The application program is configured to execute the following steps:
- receiving (i) an activation code to activate one of the medical features of the application program and (ii) one or more parameters associated with a prescription, wherein the activation code includes an identifier that identifies one of the medical features that is to be activated;
- transmitting, by the application program, the activation code via the network from the mobile device to the server;
- receiving, by the application program, an authorization code from the server, wherein the authorization code indicates a validity of the activation code; the authorization code may be received in response to the transmission of the activation code to the server;
- activating, by the application program, the one indicated medical feature using the activation code, the activation being performed in response to the activation code being valid;
- configuring, by the application program, the activated medical feature using at least one of the one or more parameters associated with the prescription; and
- generating, using the activated medical feature, data based on the one or more parameters associated with the prescription, the generated data being indicative of how the drug shall be administered;
- outputting the generated data via a user interface of the mobile device and/or sending the generated data to the apparatus for administering the drug.

A 'feature' as used herein is a piece of program logic installed on the mobile device. The piece of program logic may be based on software, firmware, hardware or any combination thereof. For example, a feature may be a function or a module the application program receiving the activation code or of another application program executed on the mobile device and interoperatively coupled to said application program. For example, the medical feature may be a piece of program logic causing the mobile device to display medical data, e.g. medical treatment advice, dosage information, diagnostic information or the like. Alternatively or in addition, the piece of program logic in activated state may be operable to cause the mobile device to send control data to a medical device such as a glucose-meter or insulin pump for controlling execution of said medical device and/or to receive measurement data from a medical device.

According to embodiments, the medical feature is a physician-prescribable feature, i.e. piece of program logic that shall be executed only in case the patient using the application program has received a prescription for the function provided by said program logic from a medical professional such as a physician. Using an activation code for physician-prescribable features may be advantageous as it may be possible to create and communicate the prescription fully automatically to a patient and in some embodiments even to fully automatically administer the drug in accordance with the parameters of the prescription to the patient.

In some embodiments, the application program is a diabetes management application. The user of the mobile device may be the owner or user of a medical device such as a glucose meter or an insulin pump.

Said features may be advantageous as a highly secure apparatus is provided that is operable to ease the application of prescribed drugs, in particular to make the selection and/or administration of the appropriate dosage more save. Outputting the generated data via a user interface of the mobile device, e.g. displaying the data on a screen of the mobile device, may enable a user to manually use an apparatus for administering the drug in accordance with the output data. In addition, or alternatively, said apparatus for administering the drug may comprise an interface to automatically receive the generated data and to automatically select and output an amount of the drug specified in the generated data and/or to automatically administer the drug. The administration of the drug may comprise administering the drug e.g. orally, intravenously and/or by simply selecting and outputting, a calculated amount of the drug to the patient to enable the patient to manually administer the drug.

Said features may be advantageous as they allow a doctor or any other kind of medical professional to automatically or semi-automatically and specifically control if a patient should have access to a particular feature of the application program running on the mobile device. This may increase the safety of using various diagnostic or treatment devices in the biomedical sector and may reduce the risk associated with displaying diagnostic data or treatment data to the patient who may react on said information inadequately. For example, a feature of the program could relate to displaying some treatment advice or diagnostic data to a patient, i.e., to the user of the mobile device. Said displayed data may induce a medical layperson e.g..to apply a recommended and displayed insulin dosage immediately. However, the patient may apply the insulin bolus without observing hygiene standards. By activating the feature by means of an activation code, it is possible to provide a patient with a medical device or application program that is deactivated at first, and that may be activated later. The activation may be performed e.g. by a health professional after having explained in detail to the patient how a particular medical device should be handled and what kind of hygiene measures should be taken. It may also be possible that some features are activated after the patient has participated in a training course for using said feature of the application program and/or in a training course for using a medical device exchanging data with said feature.

Thus, the safety of providing complex medical information and medical devices to patients may be increased.

In a further aspect, the invention may allow a decoupling of the physical provisioning of a medical device and/or medical software from the moment of enabling a user, i.e., a medical layperson, to actually use said device or software. This may be advantageous in particular for complex devices and software modules comprising a plurality of different functions which may be selectively and sequentially activated. Thus, it is possible to selectively activate functions of a medical device and/or medical software that can safely be assumed to be manageable by the user both cognitively as well as practically. Other features may be activated later after the patient has participated in a training course or has been instructed by the doctor.

According to embodiments the generated data comprises one or more of:
- bolus dosage data, the bolus dosage data being indicative of the amount of the drug to be administered;
- a treatment scheme for administering the drug;
- an indication of one or more time moments for administering the drug;
- a warning message not to administer the drug;
- a human-readable advice how to use the apparatus for administering the drug.

According to embodiments the generated data sent to the apparatus for administering the drug comprises:
- configuration data, wherein the apparatus for administering the drug is configured such that it administers the drug in an amount, in a composition and/or at a time as specified in said configuration data; and/or
- command data, wherein the apparatus for administering the drug is configured such that it administers the drug immediately upon receipt of the command data, wherein the drug is administered in an amount as specified in said command data.
For example, the control data may trigger an insulin pump to apply insulin to a patient's body, or may trigger a glucose meter to puncture the skin of a patient for obtaining blood, or the control data may trigger a calibration or cleansing of the measuring device, may trigger the processing of a test strip, or the like. The measuring device and/or the apparatus for administering the drug may only in the paired state be enabled to receive and process control data from the activated feature of the application program and/or to return measurement data selectively to the activated feature of the application program.

According to embodiments the apparatus for administering the drug comprises a processor and an interface for exchanging data with the application program. The interface is configured to pair the apparatus for administering the drug to the application program. In a paired state the apparatus for administering the drug and the application program are connected via a data communication connection. The pairing is such that only in the paired state the one medical feature of the application program, if successfully activated, is configured to send the generated data being indicative of how the drug shall be administered to the apparatus for administering the drug.

The connection established after pairing between the application program and the apparatus for administering the drug may be a WiFi connection, a Bluetooth connection, a cable connection, or any other suitable data transfer connection. Preferable, the connection is secured by a cryptographic protocol, e.g. SSH and/or TLS. If the pairing was executed successfully, the apparatus for administering the drug and the application program are in a paired state. Otherwise, both objects are not in the paired state.

According to embodiments the apparatus for administering the drug is an insulin pump and the drug is insulin.

According to embodiments the treatment apparatus further comprises a medical measurement-device. The measurement device comprises a processor and an interface for exchanging data with the application program. The interface is configured to pair the measurement device to the application program. In a paired state the measurement device and the application program are connected via a data communication connection. The pairing is such that only in the paired state the one medical feature of the application program, if successfully activated, is configured to receive measurement data from said measurement device and use said measurement data as input for the generating of the data being indicative of how the drug shall be administered. According to embodiments, the connection established after pairing between the application program and the measurement device may be a WiFi connection, a Bluetooth connection, a cable connection, or any other suitable data transfer connection. Preferable, the connection is secured by a cryptographic protocol, e.g. SSH and/or TLS.

According to embodiments, the data being indicative of how the drug shall be administered is generated by using parameters specified in the prescription in combination with measurement data gathered from the mammal by a medical measuring device as input. This may be advantageous, because a particularly accurate, reliable, individualized and safe method of determining a drug dosage that is best for an individual patient at a given time is provided. For example, the measurement data may indicate a current glucose level, and the prescription may comprise a blood glucose level range considered as optimal or healthy. By considering the current glucose level of a patient (which may vary considerably in the run of a day) as well as said "desired" blood glucose range, the amount of insulin necessary to shift the current glucose level in the appropriate concentration range may be facilitated. Thus, the apparatus may allow adapting a particular drug dosage specified in a prescription to the dynamically varying metabolomic state of a patient, thereby preventing an administering of too much or too little of the drug.

According to embodiments the measurement-device is a glucose-meter and the measurement data comprises a glucose level of the mammal.

According to embodiments the treatment apparatus further comprises a server that is configured to:
- generate the activation code;
- provide the generated activation code directly or indirectly to the application program running on the mobile device;
- store a copy of the generated activation code as a reference value;
- receive the activation code from the application program via the network;
- check if the received activation code is valid, the checking comprising comparing the received activation code with the stored reference value;
- in case the received activation code is valid, returning the authentication code to the application program.

For example, an 'activation code' may be an alphanumeric character string.

In a further aspect, a method for activating a medical feature of an application program executed on a mobile device is provided. The method comprises receiving, by the application program, (i) an activation code to activate a feature of the application program and (ii) one or more parameters associated with a prescription, where the activation code includes an identifier that identifies the feature to be activated. The method further comprises transmitting, by the application program, the activation code via a network from the mobile device to a server; and receiving, by the application program, an authorization code from the server, where the authorization code indicates a validity of the activation code. The method further comprises activating, by the application program, the feature using the activation code, the activation being performed in response to the activation code being valid. The method further comprises configuring, by the application program, the activated feature using at least one of the one or more parameters associated with the prescription. The method further comprises generating, using the activated feature, data based on the one or more parameters associated with the prescription.

In a further aspect, a method for activating a bolus advisor feature of an application program executed on a mobile device is provided. The method comprises receiving, by the application program, (i) an activation code to activate the bolus advisor feature of the application program and (ii) one or more parameters associated with a prescription, where the activation code includes an identifier that identifies the bolus advisor feature to be activated. The method further comprises transmitting, by the application program, the activation code via a network from the mobile device to a server; and receiving, by the application program, an authorization code from the server, where the authorization code indicates a validity of the activation code. The method further comprises activating, by the application program, the bolus advisor feature using the activation code, the activation being performed in response to the activation code being valid. The method further comprises configuring, by the application program, the activated bolus advisor feature using at least one of the one or more parameters associated with the prescription. The method further comprises generating, using the activated bolus advisor feature, bolus dosage data based on the one or more parameters associated with the prescription.

A 'bolus advisor feature' as used herein is a piece of program logic operable to calculate and output a bolus via a user interface, e.g. a display or speaker of the mobile device or via a printer connected to the mobile device. A 'bolus' is a single dose for administration of a particular drug, medication or other substance. The function may take several parameter values as input, e.g. physiological parameters of the patient such as gender, age, metabolite concentration in the blood or urine or the like for calculating the bolus that is outputted.

In yet another embodiment, a method for activating a medical feature, e.g. a bolus advisor feature, of an application program executed on a mobile device comprises receiving, by a healthcare professional, an activation code from a server to activate the medical feature of the application program, where the activation code includes (i) a country code of a country where the activation code is to be used, (ii) an identifier that identifies the medical feature to be activated, and (iii) an alphanumeric string. The method further comprises receiving, by the application program, (i) the activation code and (ii) one or more parameters associated with a prescription from the healthcare professional; and transmitting, by the application program, the activation code via a network from the mobile device to the server. The method further comprises receiving, by the application program, an authorization code from the server, where the authorization code indicates whether the activation code is valid. The method further comprises activating, by the application program, the medical feature using the activation code, the activation being performed in response to the activation code being valid. The method further comprises configuring, by the application program, the activated medical feature using at least one of the one or more parameters associated with the prescription. The method further comprises generating, using the activated medical feature, data that is indicative of how the drug shall be administered based on the one or more parameters associated with the prescription. For example, said generated data may be bolus dosage data.

In a further aspect, the disclosure relates to a mobile device of a user. The device comprises a processor and a storage medium. The storage medium comprises computer-interpretable instructions which encode an application program configured to perform a method for activating a medical feature of an application program according to any one of the embodiments described herein.
Further areas of applicability of the present disclosure will become apparent from the detailed description, the claims and the drawings. The detailed description and specific examples are intended for purposes of illustration only and are not intended to limit the scope of the disclosure.

### Brief description of the drawings

In the following embodiments of the invention are explained in greater detail, by way of example only, making reference to the drawings in which:
- Figure 1: is a diagram depicting a handheld glucose meter in data communication with a diabetes management application residing on a mobile phone;
- Figure 2: is a block diagram of an exemplary hardware arrangement for the glucose meter;
- Figure 3: is a sequence diagram illustrating an exemplary sequence for taking a blood glucose measure using the glucose meter;
- Figure 4: is a flowchart illustrating an exemplary technique for transmitting blood glucose measures individually from the glucose meter;
- Figure 5: is a flowchart illustrating an exemplary technique for processing glucose measures received by the diabetes management application; and
- Figure 6: shows a screenshot of a mobile phone application for diabetes care including a bolus advisor.
- Figure 7: is a flowchart illustrating an exemplary technique for activating a bolus advisor on a mobile phone application for diabetes care.
- Figure 8: is a flowchart of a method for activating a medical feature of an application program executed on a mobile device.
- Figure 9: shows a table of parameters (settings) associated with a prescription used to setup an activated feature of an application program executed on a mobile device.

The drawings described herein are, for illustrative purposes only, of selected embodiments and not all possible implementations, and are not intended to limit the scope of the present disclosure. Corresponding reference numerals indicate corresponding parts throughout the several views of the drawings.

### Detailed description

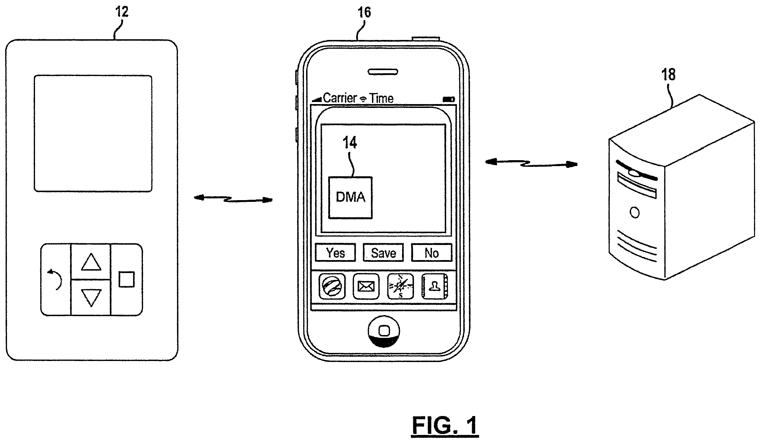

Figure 1 depicts an exemplary handheld glucose meter 12 in data communication via a wireless data link with a diabetes management application 14. The glucose meter 12 is configured to receive a sample of blood from a patient and determine a blood glucose measure for the patient from the blood sample. One or more blood glucose measures may in turn be transmitted over the wireless data link to the diabetes management application 14 for further processing. In an exemplary embodiment, the diabetes management application 14 resides on a mobile phone 16. In other embodiments, the diabetes management application 14 may be native to a remote server 18 with its user interface presented on the mobile phone 16.

The mobile phone 16 may be a handheld computing device arranged to include a hardware layer, an operating system layer, an application layer, and a user interface layer. For example, the hardware layer includes a processor and memory. The operating system layer includes an operating system. The operating system is a set of instructions stored in memory and executed by the processor. For example only, the operating system may include Android OS, iOS, or any other suitable mobile phone or tablet operating system. The application layer includes at least one software application, for example the diabetes management application 14. The user interface layer includes a user input device, such as a keyboard or a touch screen interface, and a display screen.

In some embodiments, data is transferred to and from the glucose meter 12 using the Bluetooth wireless technology standard (e.g., low energy feature of Bluetooth 4.0) although other types of communication transports are contemplated by this disclosure. For example only, data may be transferred to and from the glucose meter 12 using a WiFi network connection or a physical cable connection. In some embodiments, the glucose meter 12 is physically connected to a personal computer (PC). The glucose meter 12 transfers data over the physical connection and is stored in memory within the PC. The PC may be configured to transmit data received from the glucose meter 12 to another computing device, such as the mobile phone 16. In another embodiment, the PC may be configured to transmit data received from the glucose meter 12 to the remote server 18. The mobile phone 16 may then communicate with the remote server 18. For example, the remote server 18 may transmit data to the mobile phone 16. The mobile phone 16 stores the received data. The diabetes management application 14 may process the data stored within the mobile phone 16.

In other embodiments, the mobile phone 16 may communicate the one or more glucose measures to the remote server 18. For example, the mobile phone 16 may be configured to determine whether to communicate the one or more glucose measures to the remote server 18. Upon receiving the one or more glucose measures from the meter 12, the mobile phone 16 determines whether to communicate the one or more glucose measures to the remote server 18. In some embodiments, the mobile phone 16 is configured to automatically communicate the one or more glucose measures to the remote server 18.

In yet another embodiment, the mobile phone 16 is configured to determine whether the remote server 18 has sent a glucose measure request to the mobile phone 16. When the mobile phone 16 determines the remote server 18 has sent a glucose measure request to the mobile phone 16, the mobile phone 16 communicates the one or more glucose measures to the remote server 18. The glucose measure request may be a signal communicated over a wireless network to the mobile phone 16.

The remote server 18 stores the one or more glucose measures in memory within the remote server 18. The remote server 18 may perform further processing on the one or more glucose measures. For example, the remote server 18 associates the one or more glucose measures with other data relevant to the patient such as patient name, patient age, the date and time the one or more glucose measures were collected, patient measurement history, and any other relevant data. The remote server 18 is configured to allow remote access to data stored within the remote server 18. For example, a physician of the patient may access the one or more glucose measures stored on the remote server 18 in order to treat the patient. It is understood that while only the one or more glucose measures is discussed, the principles described herein also apply to any patient data received by the glucose meter 12.

In some embodiments, the diabetes management application 14 includes a bolus calculator. For example, the diabetes management application 14 may receive the one or more glucose measures from the glucose meter 12. The diabetes management application 14 determines a bolus calculation based on the one or more glucose measures. Further discussions of the bolus calculator can be found in the commonly assigned U.S. Pat. App. No. 13/593,593, filed August 24, 2012.

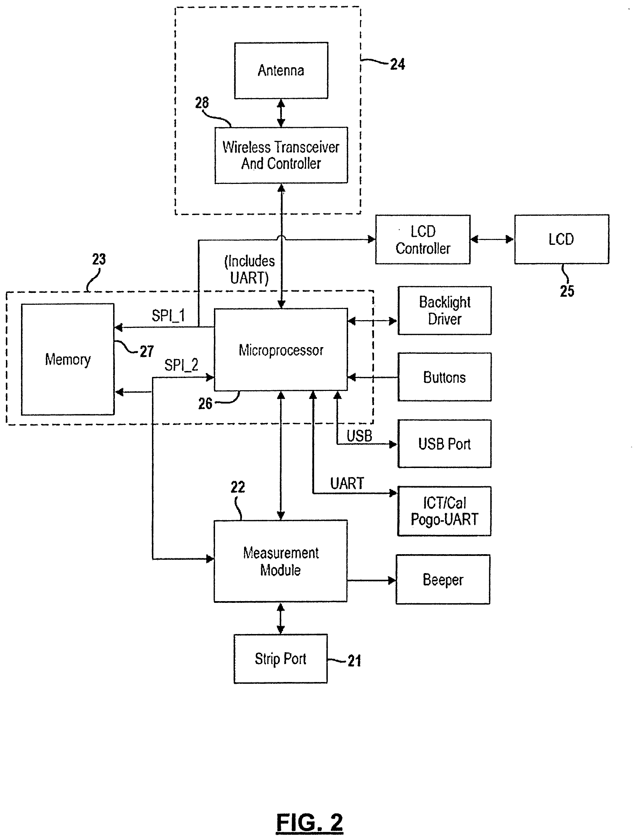

Figure 2 depicts an exemplary hardware arrangement for the glucose meter 12. The glucose meter 12 is comprised generally of a measurement module 22, a processing subsystem 23, and a communication subsystem 24. Each of these components is further described below. While the primary components are discussed herein, it is understood that other components (e.g., batteries) may be needed for the overall operation of the meter 12.

The measurement module 22 cooperatively interacts with a test strip inserted into a strip port 21 to determine a glucose measure from the sample of blood on the test strip. The measurement module 22 may include calibration information for the test strips being read by the meter 12. As used herein, the term module may refer to, be part of, or include an application Specific Integrated Circuit (ASIC); an electronic circuit; a combinational logic circuit; a field programmable gate array (FPGA); a processor (shared, dedicated, or group) that executes code; other suitable components that provide the described functionality; or a combination of some or all of the above. The term module may further include memory that stores code executed by the processor, where code, as used above, may include software, firmware, and/or microcode, and may refer to programs, routines, functions, classes, and/or objects. The processing subsystem 23 is configured to receive the glucose measures from the measurement module 22 which may in turn be stored in memory by the processing subsystem 23. Glucose measures may also be displayed by the processing subsystem 23 on a display 25. The user can interact with the meter 12 using various user interface components, such as buttons, switches, a speaker, a microphone, a USB port, etc. Each of these components is interfaced with the processing subsystem 23. In an exemplary embodiment, the processing subsystem 23 includes a microprocessor 26 and one or more volatile and/or non-volatile memories 27 although other implementations are envisioned for the processing subsystem 23.
The processing subsystem 23 is also interfaced with the communication subsystem 24. In an exemplary embodiment, the communication subsystem 24 includes a wireless transceiver 28. The wireless transceiver 28 operates to communicate the glucose measures and other data wirelessly via a data link to a remote device physically separated from the meter 12. The communication subsystem 24 can also include an antenna, a microcontroller, voltage and power control circuits, and a flash memory device. Although a few primary components of the meter 12 are discussed herein, it is readily understood that other components (e.g., power source) may be needed to implement the meter 12. The glucose measure and/or the calibration information may be used according to embodiments as measurement data that is used as input by the activated feature for generating the data being indicative of how the drug shall be administered.

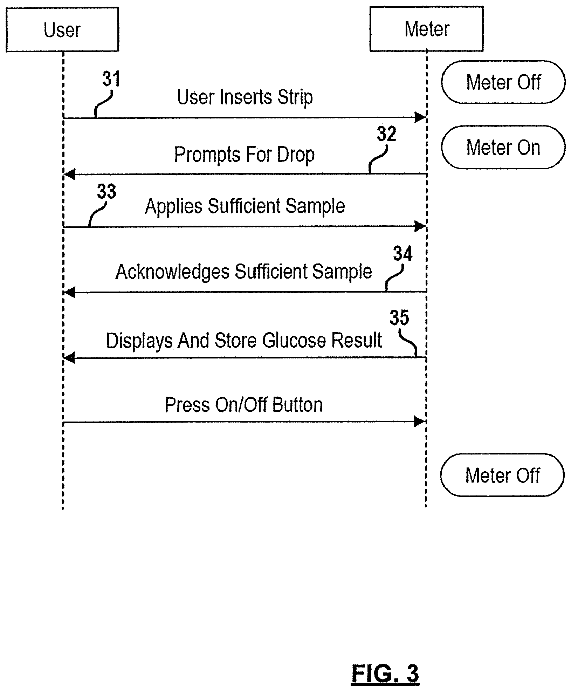

Figure 3 depicts an exemplary sequence for taking a blood glucose measure using the blood glucose meter 12. The user may insert a test strip at 31 into a port of the glucose meter 12. Insertion of the test strip prompts the glucose meter 12 to power on. The user may alternatively power on the glucose meter 12 using an on/off button. In this case, the glucose meter 12 will prompt the user to insert a test strip. The user may also power on the glucose meter 12 without inserting a test strip into the meter. In any of these cases, the glucose meter 12 may perform a quality check on the test strip inserted into the meter 12. Once the quality check has been completed, the meter 12 is ready to perform a test.

To begin a test, the user is prompted at 32 for a sample of blood. In response to the prompt, the user provides a blood sample at 33 using the test strip, where the test strip includes a reaction site that receives the blood sample from the patient. Upon receipt of the blood sample, the glucose meter 12 will proceed to analyze the blood sample in a manner readily known in the art. Before doing so, the glucose meter 12 may acknowledge the sufficiency of the blood as indicated at 34.
During the analysis, a blood glucose measure is obtained from the blood sample. The blood glucose measure will be displayed to the user and stored on the glucose meter 12 as indicated at 35. Stored glucose measures may be uploaded subsequently from the glucose meter 12 in a batch manner to a physician's computer.

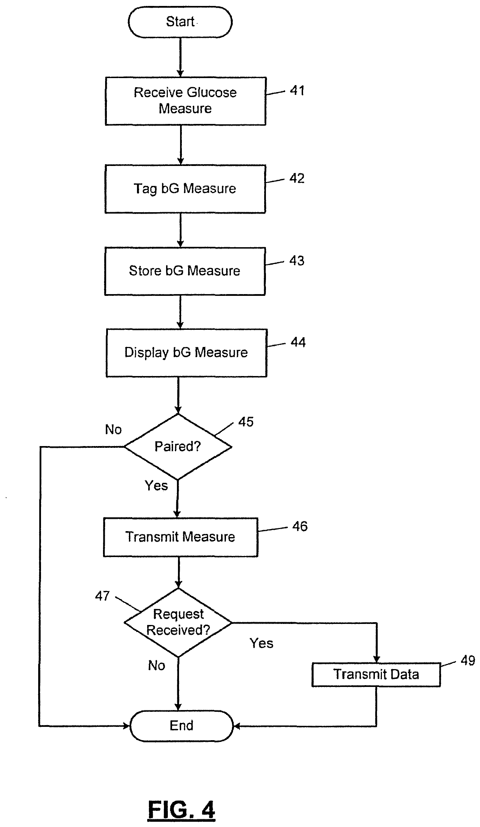

Figure 4 shows that rather than sending blood glucose measures in a batch manner, the glucose meter 12 may be configured to transmit blood glucose measures individually. The blood glucose measures may be transmitted, for example to a mobile phone, such as the mobile phone 16, or some other portable computing device carried by the user. Because the mobile phone 16 is typically in close proximity to the user, it may be used as a data collector for the patient's blood glucose measures. The diabetes management application 14 residing on the mobile phone 16 can then be used for data analysis as well as other sophisticated diabetes management functions. Consequently, the processing power and memory available on the glucose meter 12 can be streamlined, thereby reducing the cost of the glucose meter 12.

Upon determining a blood glucose measure, the blood glucose measure is first tagged at 42 with identifying information. Identifying information may include but is not limited to a timestamp for when the measure was taken, a serial number for the meter 12, and other information pertaining to the test strip. Each blood glucose measure is also tagged with a unique sequence number assigned by the glucose meter 12. In one embodiment, a counter is incremented each time a glucose measure is taken and the value of the counter is assigned to the blood glucose measure. The sequence number may be used to retrieve missing data from the glucose meter 12 as is further described below. Once tagged, the blood glucose measure is stored at 43 in a memory of the glucose meter 12 and displayed to the user at 44 on a display of the glucose meter 12.

Next, the glucose meter 12 determines at 45 whether it is paired via a wireless data link with another device, such as the mobile phone 16. The current blood glucose measure is transmitted at 46 to the mobile phone 16 when the glucose meter 12 is paired to the mobile phone 16. The glucose meter 12 may be configured to automatically transmit the blood glucose measure to the mobile phone 16. Alternatively, the person with diabetes may operate the glucose meter 12 manually to transmit the blood glucose measure to the mobile phone 16. While reference is made throughout this disclosure to a message being sent with a single glucose measure, it is envisioned that in some embodiments the message transmitted by the glucose meter 12 can contain one or more glucose measures.

In one embodiment, the blood glucose measure is transmitted automatically and without user intervention. For example, after taking a glucose measure, the glucose measure is transmitted automatically after a predefined timeout period (e.g., five seconds) without receiving any input from the user. In another embodiment, the blood glucose measure is transmitted automatically in response to the user navigating away from the measurement result screen. In a similar manner, the blood glucose measure may be transmitted automatically in response to the meter 12 being powered down by the user. It is envisioned that the mobile phone 16 and/or the diabetes management application 14 is authenticated with the glucose meter 12 during the pairing process.

The glucose meter 12 may also receive a request for missing glucose measures at 47 from the diabetes management application 14. In one embodiment, the request identifies any missing glucose measures by its sequence number as will be further described below. In response to receiving a request, the glucose meter 12 will transmit the missing glucose measures at 49 to the diabetes management application 14. It is to be understood that only the relevant steps are discussed in relation to Figure 4 and that other software-implemented instructions may be needed to transmit data from the glucose meter 12. In an exemplary embodiment, the method described above is implemented by a user interface module residing on the glucose meter 12.

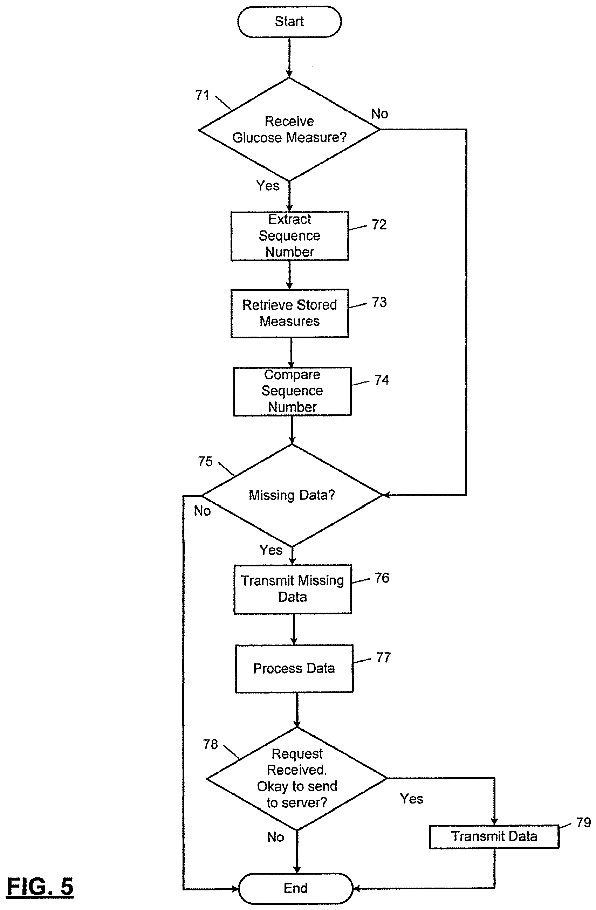

Figure 5 depicts an exemplary method for processing glucose measures received by the diabetes management application 14 residing on the mobile phone 16. In the exemplary embodiment, glucose measures are transmitted individually to the diabetes management application 14 as described in relation to Figure 4. It is envisioned that other techniques for transmitting the glucose measure to the diabetes management application 14 are contemplated by this disclosure. For example, the glucose meter 12 may transmit the glucose measures via a WiFi connection, a Bluetooth connection, a cable connection, or any other suitable data transfer connections and/or protocols.

Upon receiving a glucose measure at 71, a sequence number associated with the glucose measure is first determined by the diabetes management application 14. A unique sequence number is assigned by the glucose meter 12 to each glucose measure as described above. Thus, the sequence number associated with the glucose measure can be extracted at 72 from the data packet or message received from the glucose meter 12. In some embodiments, a series of glucose measures previously received from the glucose meter 12, along with their associated sequence numbers, may be stored in a memory device contained in or coupled to the mobile device and thus be accessible to the diabetes management application 14. In other embodiments, only the most recently received glucose measure and its sequence number is stored by the diabetes management application 14. In either case, the stored glucose measure(s) along with associated sequence number(s) are retrieved from memory.

A comparison is made at 74 between the sequence number extracted from the present glucose measure and the sequence numbers of the stored glucose measures. A request for missing glucose measures is transmitted by the diabetes management application 14 to the glucose meter 12 at 76 when an omission in the sequence is detected. For example, a request for missing glucose measures is transmitted when the extracted sequence number is 84 and the highest stored sequence number is either 81 or 82. Conversely, a request is not transmitted when the extracted sequence number is 84 and the highest stored sequence number is 83. Because this comparison is made for each glucose measure received by the diabetes management application 14, a comparison of the extracted sequence number only needs to be made to the highest stored sequence number. In other embodiments, the diabetes management application 14 may analyze the series of glucose measures for omitted measures and send a request for each glucose measure missing from the series of glucose measures. The request for missing glucose measures can be transmitted in accordance with the protocol described in relation to Figure 1.

Said features may be advantageous as they may allow a user to continuously and centrally collect a complete series of glucose measures on his mobile device even in case some of the glucose measures were performed in the absence of the mobile device, e.g. in the absence of a mobile phone. In praxis, there exist multiple situations when a glucose measurement is taken but the mobile device may not be available: the mobile phone may have run out of power, the patient may be located in a building or plane where the use of a mobile phone is forbidden or the patient may simply have forgotten to carry his mobile phone with him. The patient may forget to transfer the glucose level measurement data to his mobile phone later, resulting in a fragmentary documentation of his glucose level and thus to a lower quality of the collected data that forms the basis for subsequent diagnoses and bolus calculations. By automatically assigning a sequence number by the glucose meter in combination to the above described evaluation of the sequence numbers for completeness, missing measurement values may be automatically transferred to the mobile device of the patient later, thereby ensuring a complete and continuous documentation of the measured glucose level of the patient.

At 77 the diabetes management application 14 processes the glucose measures. For example, the diabetes management application may correlate the most recently received glucose measure with previously received glucose measures. The diabetes management application 14 may then generate a graphical representation of the glucose measures. The diabetes management application 14 displays the graphical representation on a screen of the mobile phone 16. The diabetes management application 14 may receive a request to transmit the glucose measure(s) to a remote location, such as the remote server 18.

At 78 the diabetes management application 14 determines whether a request was received to transmit the glucose measure(s) to the remote server 18. When the diabetes management application 14 determines that a request was received, the diabetes management application 14 transmits the glucose measure(s) to the remote server 18. Transmitting the glucose measure(s) may include packaging the glucose measure(s) in a packet configured to be received and interpreted by the remote server 18 at 79. In another embodiment, the diabetes management application 14 automatically transmits the glucose measure(s) to the remote server 16 upon receiving the glucose measure(s) from the meter 12.

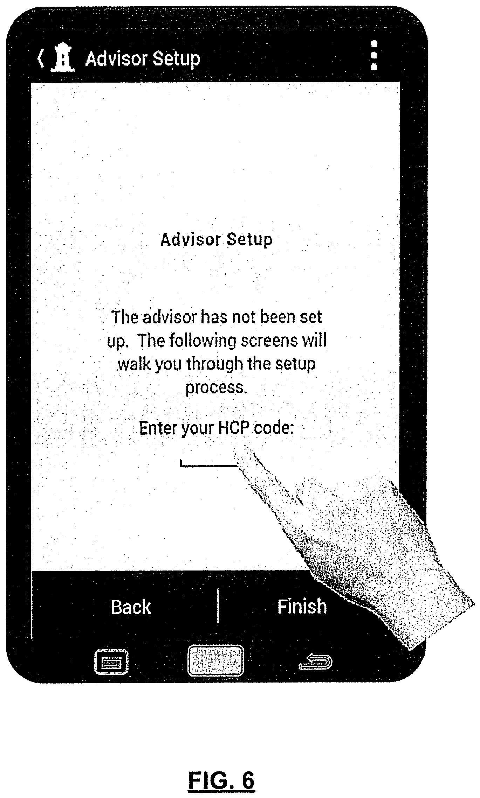

Figure 6 shows a screenshot of the diabetes management application 14 according to the present disclosure including a bolus advisor that is downloaded on the mobile phone 16 as part of the application but not activated due to medical safety concerns. To address the medical safety concerns, the person with diabetes must receive guidance from a healthcare professional prior to receiving an activation code to activate the bolus advisor. The person with diabetes enters the activation code received from the healthcare professional into the application to gain access to the bolus advisor. The activation key concept disclosed herein with reference to the bolus advisor feature of the application can also be applied to other medical features of the application such as a carbohydrate counter and insulin pump control, for example.

To obtain the activation key, the person with diabetes may send an email message or SMS to a healthcare professional using the mobile phone 16. The email message or the SMS may include information specific to the mobile phone 16. The information specific to the mobile phone may be used to generate an activation key specifically for the mobile phone 16. Alternatively, the healthcare professional may provide the activation key to the person with diabetes in person.

The healthcare professional may have an application that generates the activation key with constraints such as a time limitation for using the activation key that is provided to the person with diabetes. The person with diabetes uses the activation key to activate the bolus advisor and then enters a bolus advice parameter provided by the healthcare professional into the diabetes management application 14. The diabetes management application 14 then calculates bolus. Based on the calculated bolus, an insulin pump, which communicates with the mobile phone 16 via the diabetes management application 14, administers insulin to the person with diabetes. Typically, bolus calculators require a prescription. Use of a written prescription, however, may create an administrative burden to the healthcare professional and may inconvenience the person with diabetes since such a requirement would erode the usefulness and flexibility offered by the diabetes management application 14, which is typically downloaded from an app store to the mobile phone 16. Instead, the healthcare professional transmits to the person with diabetes an activation key to activate the bolus advice feature and the necessary parameters for the bolus advice feature. The person with diabetes enters the bolus activation key into the diabetes management application 14 on the mobile phone 14 and then accesses the bolus advice feature of the diabetes management application 14.

The person with diabetes can activate the bolus advice feature of the diabetes management application 14 using a simple setup provided by the diabetes management application 14. The setup may include inputting information such as bG target range, meal rise, insulin to carbs ratio, insulin to bG (sensitivity) ratio, and maximum bolus. Other parameters may use default values. The diabetes management application 14 may use two separate screens to allow the person with diabetes to enter the activation code and to input the information into the simple setup. The diabetes management application 14 can enable or disable the need for the activation key to activate the bolus advisor feature to meet the needs of various countries.
The diabetes management application 14 may run on a variety of mobile devices such as Android-based phone, iPhones, and/or Windows phones. The diabetes management application 14 can run on smartphones, tablets, as well as other portable computing devices. The diabetes management application 14 can support core functionality (data entry, reminders, structured tests, and simple reporting) when disconnected from the cellular network and the Internet. When network access is available, the diabetes management application 14 can allow data sharing with healthcare professionals and caregivers via the mobile device communication system or a web account data sharing system. The diabetes management application 14 can also support connectivity to a Bluetooth Low Energy (BLE) bG Meter and other bG meters via BLE/BT transport.

The purpose of the diabetes management application 14 is to make it easier for patients to provide reliable health information to their caregivers and healthcare professionals for the purpose of helping the patient improve their ability to manage their diabetes. The diabetes management application 14 is intended for use by individual persons with diabetes. The diabetes management application 14 can support entry, transfer, storage, display, and analysis of blood glucose data and other related health indicators. As people generally have their mobile devices with them all the time, the diabetes management application 14 application can provide persons with diabetes an always-available and easy-to-use way to log and share their health information.

The diabetes management application 14 can support data sharing by means of SMS and MMS messages, by email delivery of reports, and by data upload to a web-based diabetes management system. These options can make patient data available to the patient's caregivers and healthcare professionals, improving their ability to help the patient manage their diabetes.

The diabetes management application 14 can support a plurality of languages. The diabetes management application 14 can support additional structured test protocols, integration of a food database, and transfer of data from other BT/BLE-enabled health devices such as blood pressure cuffs, weight scales, and so on.
While the code of the diabetes management application 14 may differ to the extent that it can support different mobile devices running different operating systems, the diabetes management application 14 provides a common platform for diabetes management in at least the following senses: The diabetes management application 14 allows easy addition of new languages, includes clean separation of core logic from user interface functions, includes clean separation of core logic from data access mechanisms, and supports addition of new features.

The user interface of the diabetes management application 14 can be delivered both by the diabetes management application 14 and by content hosted on a web-based diabetes management system. The diabetes management application 14 can include external system interfaces to: export and import of data and settings from the web-based diabetes management system; receive data from supported BT/BLE Meter; email, MMS, and SMS text messaging (when available); backup and restore data from via email; export XML file to the web-based diabetes management system; and 3rd party data analytics service.

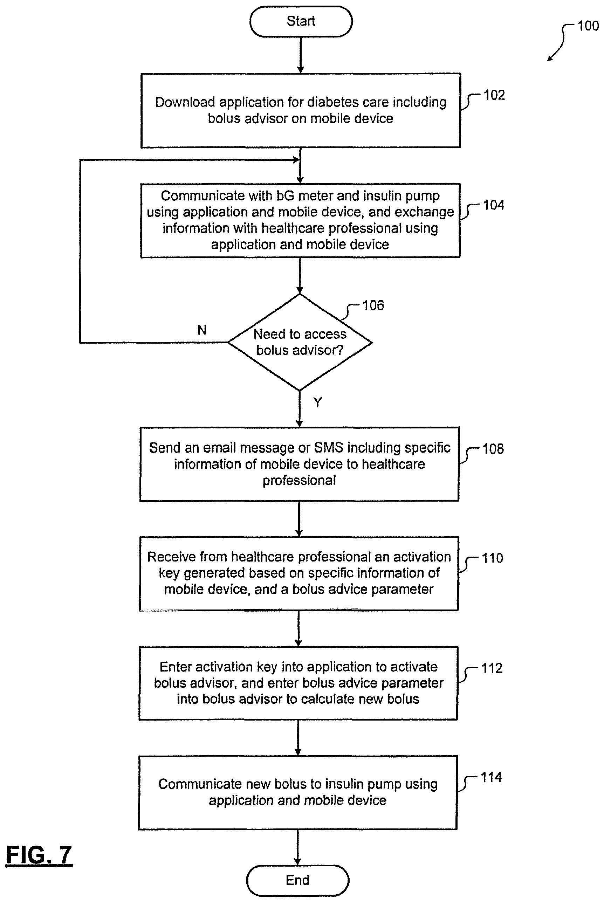

Figure 7 is a flowchart illustrating an exemplary technique for activating a bolus advisor feature on a mobile phone application for diabetes care. At 102, the person with diabetes downloads an application such as the diabetes management application 14 on a mobile device such as the mobile phone 16. The application includes among other things a bolus advisor. In addition, the application may include other medical features such as a carbohydrate counter and insulin pump control. The teachings of the present disclosure, while disclosed with reference to the bolus advisor feature, can also be applied to other medical features such as the carbohydrate counter, the insulin pump control, and so on.

At 104, the person with diabetes may communicate with a blood glucose meter and/or an insulin pump using the application and the mobile device. For example, the person with diabetes may perform a blood glucose test using the blood glucose meter and may gather data related to the blood glucose test from the blood glucose meter using the application and the mobile device. In addition, the person with diabetes may control and/or test the blood glucose meter and/or the insulin pump using the application and the mobile device.

At 106, the person with diabetes may decide whether to access the bolus advisor in the application. If accessing the bolus advisor is unnecessary, the person with diabetes may continue to use the application and the mobile device to communicate with the blood glucose meter and/or the insulin pump as described above. In addition, the person with diabetes may continue to use the application and the mobile device to communicate with the healthcare professional. If, however, the person with diabetes needs to access the bolus advisor in the application, the person with diabetes may send an email message or SMS to a healthcare professional at 108. The email message or the SMS may include specific information of the mobile device. For example, the specific information of the mobile device may include but is not limited to a serial number of the mobile device, an identifier associated with a processor (e.g., CPU ID), or an identifier associated with a communication device (e.g., a MAC address), and so on. In addition, the email message or the SMS may include data gathered by the application and the mobile device from the blood glucose meter and/or the insulin pump.

At 110, the person with diabetes may receive from the healthcare professional an email message or SMS that includes an activation key generated by the healthcare professional based on the specific information of the mobile device. In addition, the email message or SMS may include a bolus advice parameter generated by the healthcare professional based on the data from the blood glucose meter sent by the person with diabetes. For example, the bolus advice parameter may include but is not limited to one or more of bG target range, meal rise, insulin to carbs ratio, insulin to bG (sensitivity) ratio, and maximum bolus. The email message or SMS may also include diagnostic and/or calibration information for the blood glucose meter and/or the insulin pump.

At 112, the person with diabetes may enter the activation key into the application to activate the bolus advisor. In addition, the person with diabetes may enter the bolus advice parameter into the bolus advisor feature of the application to calculate new bolus. At 114, the application in the mobile device may communicate the new bolus to the insulin pump using the mobile device. Subsequently, the insulin pump may administer the new bolus to the person with diabetes.

In some implementations, the healthcare professional may include the bolus advice parameter in the activation key itself so that the person with diabetes has to enter only the activation key into the application, and on entering the activation key, the bolus advisor automatically configures itself based on the bolus advice parameter included in the activation key. This can ensure that the person with diabetes cannot alter the bolus advice parameter. Additionally, each time the bolus advice needs to be changed, the person with diabetes will be required to request and acquire a new activation key. The bolus advice parameter and the bolus calculated based on the bolus advice parameter will remain valid and in effect for the duration for which the activation key remains valid or until a new activation key is obtained, whichever occurs first.

In some implementations, the application may verify whether the activation key received is in fact from a trusted source. For example, the verification may be performed using a hashing algorithm included in the application. The healthcare professional may encrypt the activation key using the same hashing algorithm used by the application. For example, the activation key may be generated by hashing identifying information (e.g., name, patient ID, and so on) of the person with diabetes, an identifier associated with the mobile device received via email or SMS message from the person with diabetes, etc.

The application uses the hashing algorithm to decrypt the activation key when entered by the person with diabetes. The hashing algorithm in the application compares the decryption result to the identifying information entered into the application by the person with diabetes and/or the identifier associated with the mobile device. If the decryption result matches the identifying information of the person with diabetes and/or the identifier associated with the mobile device, the application determines that the activation key is received from a trusted source, and the application grants access to the bolus advisor. If the decryption result does not match the identifying information of the person with diabetes and/or the identifier associated with the mobile device, the application determines that the activation key is not received from a trusted source, and the application does not grant access to the bolus advisor.

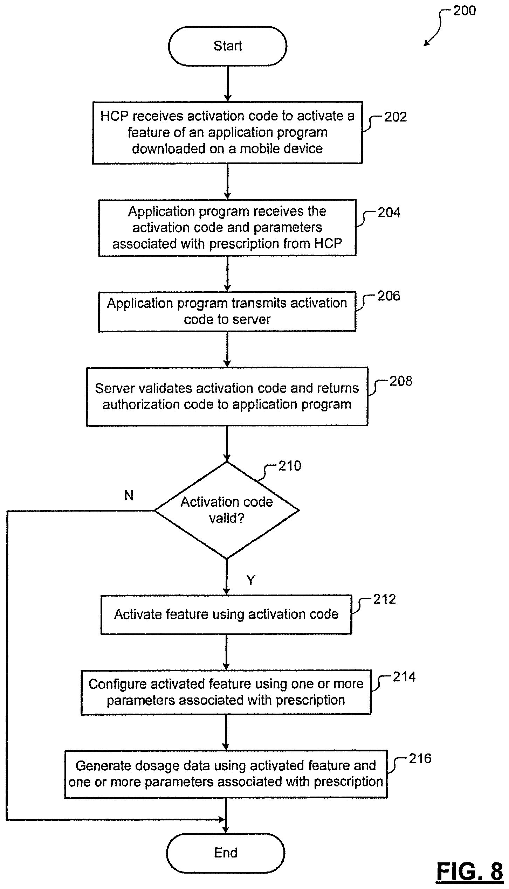

Figure 8 is a flowchart of a method 200 for activating a medical feature of an application program executed on a mobile device. The application program is downloaded by a patient (e.g., a person with diabetes) on the mobile device. The application program includes one or more features that require a prescription from a healthcare professional (e.g., a physician) to activate and use the feature. For example, the feature may include a bolus advisor feature.
At 202, the physician may obtain an activation code to activate the feature. For example, the physician may log into a server that generates and distributes activation codes. The physician may request an activation code to activate a feature of the application program used by a patient. The server may verify the credentials of the physician and provide the activation code to the physician. For example, the server may provide the activation code in a printed form or electronically (e.g., via an email).

Each activation code may be unique (i.e., different) and may not be repeated. Each activation code may include an identifier that identifies the feature to be activated, a country code of the country where the activation code is to be used, and an alphanumeric string. For example, the identifier may include one numeral, the country code may include two characters, and the alphanumeric string may include four characters. The activation code may have an expiration date. For example, if the prescribing physician requires the person with diabetes to return in six months to evaluate and modify the bolus advice parameters, the activation could have a time limit where it stops working after the set time. In other words, the activation code can remain valid for a predetermined time period and can expire at an end of the predetermined time period.

The physician may then provide the activation code and one or more parameters associated with the prescription to the patient (e.g., via an SMS, an email, or a telephone call). The parameters are used to control treatment of the patient through the feature of the application program and are therefore prescribed by the physician.

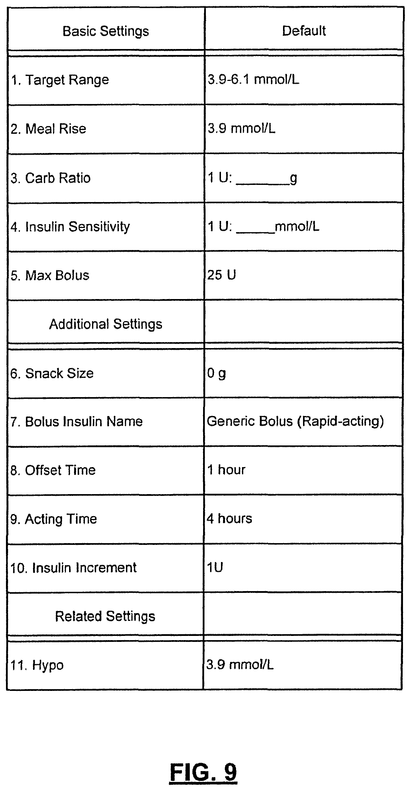

Some of the parameters may be paired (i.e., tied) to the activation code and may be used to setup the feature once activated.
"Setting up a feature" by means of one or more parameters as used herein means that the parameters are used to configure the feature upon activation of the feature. Parameters being "paired with the activation code" are parameters that are communicated to the application program as part of the activation code or which are communicated together with the activation code or are otherwise linked or stored in association with a particular activation code.
Other parameters may be used by the feature subsequent to the activation and setup procedure to generate dosage data. For example, said parameters may be used as input for generating the data being indicative of how the drug shall be administered.
For example, Figure 9 shows a table that includes settings (i.e., parameters used for setting up the feature) that the physician may be required to prescribe to a patient with diabetes. The parameters listed under Basic Settings are used in the basic setup of the bolus advisor feature of the application program. These parameters may be paired (i.e., tied) to the activation code, for example, and may be received by the application program 14 together with the activation code. The parameters listed under Additional Settings can be adjusted using a Settings/Advisor menu of the feature or the application program.
At 204, the application program receives the activation code and one or more parameters associated with the prescription from the physician. At 206, the application program transmits the activation code from the mobile device to the server via a network for validation. At 208, the server may verify one or more of the following in order to determine whether the activation code is valid. For example, the server may verify whether the activation code is received from the application program authorized to use the activation code and not from any other unauthorized application program. The server may verify whether the activation code is to be used in the country where the application program is being used. The server may verify whether the activation code has been used before. The server may verify whether the activation code has been disabled or deleted as a result of being lost or stolen.
If one or more of the above is verified, the server may determine that the activation code is valid and generate an authorization code. The authorization code may include the country code indicating the country in which the activation code is to be used, the identifier that identifies the feature to be activated using the activation code, the activation code, a status indicator indicating whether the activation code is valid, and a time stamp indicating the date and time when the activation code is validated.

In some implementations, the authorization code may include the activation code, a time stamp including the date and time when the activation code is validated, the identifier of the feature to be activated using the activation code, and an authorization indicating that the activation code is valid. The authorization may include an encrypted string obtained by concatenating the time stamp, the identifier, and the country code, followed by performing hashing and encryption functions. Additional or other security mechanisms may be used to ensure security and integrity of the activation code. The server may return the authorization code to the application program via the network.

An example of an activation code may be "EPIMcnMZ7283hlasi7d8w", wherein:
- the first and second position comprising characters "EP" may act as feature identifier,
- the third and fourth position comprising characters "IM" may act as identifier of a country in which the patient and the physician reside,
- the fifth and all following positions "cnMZ7283hla..." may be a random string or a random number created by the server.

For example, a physician may register at the server once. Then, in order to activate a medical feature of the application program of one of his or her patients, the physician may authenticate at the server and trigger the generation of an activation code comprising a new random string; for example, the physician may select one out of a plurality of features provided by the application program from a drop-down list of a GUI generated by the server. Said selection determines the feature-ID encoded in the first and second position of the activation code to be generated. The generated activation code is stored as reference value by the server. A copy of the generated activation code is returned to the physician, e.g. via an e-mail or by immediately displaying the generated activation code to the physician via a GUI. The physician may examine the patient and instruct the patient how to use bolus information or how to use a particular medical device. Afterwards, the physician may forward the copy of the activation code to the patient, e.g. by e-mail or by means of a print-out. The patient may enter the received activation code into the application program on his mobile device.

In other embodiments, the generated activation code is sent directly from the server to the application program running on the user's mobile device. The generated activation code may be sent via a network such as the internet. For example, the user may register at the server and immediately receive an activation code for some basic functionalities of the application program. Said basic functionalities may relate to managing address data and/or medical data already stored on the server or on the mobile device of the user.

According to embodiments, the generated activation code comprises a start and/or an end time, wherein the start time indicates the start time of validity of the activation code and the end time indicates when the generated activation code expires and will be considered as invalid by the server. The start time may be a current time of creating the activation code by the server or the time of receiving a request from the physician to generate the activation code. The start and end time may be taken by means of a timestamp functionality provided by an operating system of the server, e.g. Unix OS.

According to embodiments, the application program and the server establish an SSL/TLS tunnel.

The application program may be operable to access the following parameters: activation code, feature-ID, prescription parameters and further optional parameters. The parameters may have been received e.g. as user-input from a user via a GUI or may have been received via a network interface.
The application program may execute the following steps for creating and sending the activation request:
- The application program determines a current time;
- The application program generates a concatenated value CV1: CV1= concat(activation-code+prescription-parameter(s)+feature-ID+currentTime) ;
- the application program generates a hash value HV1 by: HV1=hash(CV1).
- The application program is operable to access a private signature key pK_{AP-} stored in a storage medium of the mobile device; the application program signs the hash value HV1 by using said private key:
   HV1SIG=signWithpK_{AP}(HV1); the corresponding public key pubK_{AP} (which forms an asymmetric cryptographic key pair together with pK_{AP)} may be transferred to the server, e.g. directly or by means of a key server; HV1SIG may have assigned a certificate;
- The application sends an activation request to the server; the activation request comprises the following data values: activation-code+prescription-parameter(s)+feature-ID+currentTime+HV1SIG. Thus, the activation request comprises - among other parameters - the activation code that is sent to the server.

Server receives the activation request from the application program running on the mobile device and executes the following steps:
- Verifying the signature of HV1SIG using public key pubK_{AP} by decrypting HV1SIG; the result value of said decryption step is referred to as HV1'
- calculating a hash value HV2 from the parameters (activation-code+prescription-parameter(s)+feature-ID+currentTime+HV1SIG) it received.
- comparing the decrypted HV1SIG (which is HV1) with HV2
- executing a signature check of the received HV1SIG by means of a certificate chain checking; this may allow the server to ensure that the activation code was received from a trusted application program, not by a software application of unknown origin which might calculate bolus information wrongly;
- If HV1 and HV2 are identical and if the signature of HV1SIG is valid, the server returns an authentication code to the application program; the authentication code or the message comprising the authentication code may comprise the feature-ID; otherwise, the server returns an error message to the application program.

If the application program receives the authentication code, the server activates the feature identified by the feature-ID. If the application program receives the error message, it may display the error message on the screen of the mobile device.

In the above example, the currentTime may be used as a NONCE (i.e., "number used only once"). Using a NONCE for calculating the hash value HV1 may make the method more robust against that attacker resending that valid message ("replay attacks"), and also make it a bit more complicated to derive the private key after observing a plurality of them.

The transfer of the activation code and the authentication code or the error message may be executed via the secure communication channel, e.g. by means of the SSH or SSL protocol.

The server and the application program may share the same concatenation function and the same hash algorithm. This may ensure that the hash values HSV1 and HSV2 may match and may be used as an anchor of trust between the server and the application program. This anchor of trust may be used to ensure that sensitive measurement data is transmitted from the application program to the server only in case both entities recognize each other as trustworthy: only in case the HSV1/HSV2 values match, the server will send an authentication code to the application program for activating the feature. This may ensure that the authentication code is not sent to an application program that was not thoroughly tested or that is from unknown origin. This may allow to prevent activation of features of an application program that may calculate bolus information wrongly, and thus might be a health risk for the patient.

The server will compare the received activation code with the stored reference value and will check if the activation code has meanwhile been invalidated, e.g. because the code has been reported to have been stolen. If the comparison with the reference value and the checking returns that the activation code is valid, the server will return an authentication code that automatically activates the feature identified by the feature identifier 'EP'. The server may comprise some checking logic when evaluating the activation code, whereby said checking logic is country-specific. This may be advantageous as some countries may forbid the activation of some features under any circumstances while other countries may allow activation of said feature without any legal restrictions.

The server compares the received activation code with the stored reference value and checks if the received activation code is invalid, e.g. because the code has been reported as having been stolen. If the comparison with the reference value and the checking returns that the activation code is valid, the server will return an authentication code that automatically activates the feature identified by the feature identifier 'EP'. Said features may be advantageous as said checking may be executed whenever a user tries to access and use a particular feature of the application program. The server-side checking involving a comparison of the activation code with a "blacklist" of codes reported as having been stolen increases the security as it is possible to deactivate an activation code also after its issuance.

The server may comprise some checking logic when evaluating the activation code, whereby said checking logic may be country-specific. This may be advantageous as some countries may forbid the activation of some features under any circumstances while other countries may allow activation of said feature without any legal restrictions.

In case the server determines that the received activation code is valid, the server will send an authentication code to the application program. In some embodiments, the authentication code is signed by the first private key, and the signed authentication code is returned together with a certificate that enables the application program to verify the validity of said certificate by means of a certificate chain analysis. Said features may be advantageous as they may enable the application program to make sure that the authorization code is received from a trustworthy server.

At 210, the application program checks whether the authorization code received from the server indicates that the activation code is valid.

At 212, if the authorization code indicates that the activation code is valid, the application program activates the feature using the activation code. At 214, the application program uses some of the parameters associated with the activation code, which are received with the activation code from the physician, to perform an initial setup or configuration of the activated feature. At 216, the activated feature generates dosage data according to the prescription using other parameters received with the activation code from the physician.

Following are examples of parameters that may be tied to the activation code and that may be used to perform an initial setup or configuration of the bolus advisor feature: a target range, which includes acceptable upper and lower blood glucose levels when fasting or before a meal; meal rise, which accommodates an expected rise in blood glucose levels in response to food intake; a carb ratio, which is an amount of insulin necessary to account for a specified amount of carbohydrates; an insulin sensitivity, which is an amount of insulin necessary to lower blood glucose levels by a specified amount; and a maximum bolus, which is a maximum amount of insulin to be delivered at one time.

Following are examples of additional parameters that may be used to generate bolus dosage data: a snack size, which defines a threshold of carbohydrates above which a meal rise is triggered; an offset time, which is a length of time after a bolus is administered until a reduction in blood glucose levels begins; an acting time, which is a length of time insulin is expected to be effective at lowering blood glucose levels; insulin increment, which a unit or a fraction of a unit of insulin; and hypo, which is a setting (i.e., a blood glucose concentration threshold) below which blood glucose levels are considered hypoglycemic.
The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

The techniques described herein may be implemented by one or more computer programs executed by one or more processors. The computer programs include processor-executable instructions that are stored on a non-transitory tangible computer readable medium. The computer programs may also include stored data. Nonlimiting examples of the non-transitory tangible computer readable medium are non-volatile memory, magnetic storage, and optical storage.

Some portions of the above description present the techniques described herein in terms of algorithms and symbolic representations of operations on information. These algorithmic descriptions and representations are the means used by those skilled in the data processing arts to most effectively convey the substance of their work to others skilled in the art. These operations, while described functionally or logically, are understood to be implemented by computer programs. Furthermore, it has also proven convenient at times to refer to these arrangements of operations as modules or by functional names, without loss of generality.

Unless specifically stated otherwise as apparent from the above discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage, transmission or display devices.

Certain aspects of the described techniques include process steps and instructions described herein in the form of an algorithm. It should be noted that the described process steps and instructions could be embodied in software, firmware or hardware, and when embodied in software, could be downloaded to reside on and be operated from different platforms used by real time network operating systems. The present disclosure also relates to an apparatus for performing the operations herein. This apparatus may be specially constructed for the required purposes, or it may comprise a general-purpose computer selectively activated or reconfigured by a computer program stored on a computer readable medium that can be accessed by the computer. Such a computer program may be stored in a tangible computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each coupled to a computer system bus. Furthermore, the computers referred to in the specification may include a single processor or may be architectures employing multiple processor designs for increased computing capability.

## Claims

1. A treatment apparatus comprising:
- a glucose-meter (12);
- an apparatus for administering a drug to a mammal; and
- a mobile device (16);
wherein the mobile device is connected to a server (18) via a network; wherein the mobile device hosts a diabetes management application (14); wherein the diabetes management application comprises one or more medical features, wherein a medical feature is a piece of program logic causing the mobile device to display medical data;
wherein the application program is configured to execute the following steps:
- receiving (i) an activation code to activate one of the medical features of the diabetes management application and (ii) one or more parameters associated with a prescription, wherein the activation code is entered by a person with diabetes into the diabetes management application and includes an identifier that identifies one of the medical features that is to be activated;
- transmitting, by the diabetes management application, the activation code via the network from the mobile device to the server (18);
- receiving, by the diabetes management application, an authorization code from the server, wherein the authorization code indicates a validity of the activation code;
- activating, by the diabetes management application (14), the one indicated medical feature using the activation code, the activation being performed in response to the activation code being valid;
- configuring, by the diabetes management application, the activated medical feature using at least one of the one or more parameters associated with the prescription; and
- generating, using the activated medical feature, data based on the one or more parameters associated with the prescription, the generated data being indicative of how the drug shall be administered;
- sending the generated data to the apparatus for administering the drug;
- wherein the glucose-meter comprises a processor and an interface for exchanging data with the diabetes management application (14), the interface being configured to pair the glucose-meter to the diabetes management application;
- wherein in a paired state the glucose-meter and the diabetes management application (14) are connected via a data communication connection; and whereby the pairing is such that only in the paired state the one medical feature of the diabetes management application, if successfully activated, is configured to receive measurement data from said glucose-meter and use said measurement data as input for the generating of the data being indicative of how the drug shall be administered, the measurement data comprising a glucose level of the mammal;
- wherein the glucose-meter is configured to assign a unique sequence number to each blood glucose measure, to tag each glucose measure with a time stamp and a serial number for the glucose meter, to transmit each blood glucose measure individually to the mobile device, to store the blood glucose measures in a memory of the glucose meter and to displayed to the user on a display of the glucose meter; and
- wherein the diabetes management application is configured to determine, upon receiving a glucose measure, a sequence number associated with the received glucose measure; to compare the determined sequence number with the sequence numbers of a series of glucose measures previously received from the glucose meter (12); and to transmit a request for missing glucose measures to the glucose meter when an omission in the sequence is detected; and
- wherein the glucose meter is configured to receive a request for missing glucose measures from the diabetes management application, the request identifying any missing glucose measures by its sequence number, and to transmit, in response to receiving said request, the missing glucose measures to the diabetes management application.

2. The treatment apparatus of claim 1, wherein the generated data comprises one or more of:
- bolus dosage data, the bolus dosage data being indicative of the amount of the drug to be administered;
- a treatment scheme for administering the drug;
- an indication of one or more time moments for administering the drug;
- a warning message not to administer the drug;
- a human-readable advice how to use the apparatus for administering the drug.

3. The treatment apparatus of any one of the previous claims, wherein the generated data sent to the apparatus for administering the drug comprises:
- configuration data, wherein the apparatus for administering the drug is configured such that it administers the drug in an amount, in a composition and/or at a time as specified in said configuration data; and/or
- command data, wherein the apparatus for administering the drug is configured such that it administers the drug immediately upon receipt of the command data, wherein the drug is administered in an amount as specified in said command data.

4. The treatment apparatus of any one of the previous claims,
- wherein the apparatus for administering the drug comprises a processor and an interface for exchanging data with the diabetes management application; and
- wherein the interface is configured to pair the apparatus for administering the drug to the diabetes management application (14);
- wherein in a paired state the apparatus for administering the drug and the diabetes management application (14) are connected via a data communication connection; and
- whereby the pairing is such that only in the paired state the one medical feature of the diabetes management application, if successfully activated, is configured to send the generated data being indicative of how the drug shall be administered to the apparatus for administering the drug.

5. The treatment apparatus of any one of the previous claims, wherein the apparatus for administering the drug is an insulin pump and wherein the drug is insulin.

6. The treatment apparatus of any one of the previous claims, further comprising:
- a server;
whereby the server is configured to:
- generate the activation code;
- provide the generated activation code directly or indirectly to the diabetes management application (14) running on the mobile device;
- store a copy of the generated activation code as a reference value;
- receive the activation code from the diabetes management application via the network;
- check if the received activation code is valid, the checking comprising comparing the received activation code with the stored reference value;
- in case the received activation code is valid, returning the authentication code to the diabetes management application (14).

7. A method for activating a medical feature of an diabetes management application (14) executed on a mobile device (16) and on a glucose meter, the method comprising:
- receiving, by the diabetes management application, (i) an activation code to activate the medical feature of the diabetes management application and (ii) one or more parameters associated with a prescription, wherein the activation code is entered by a person with diabetes into the diabetes management application and includes an identifier that identifies the medical feature to be activated;
- transmitting, by the diabetes management application, the activation code via a network from the mobile device to a server (18);
- receiving, by the diabetes management application, an authorization code from the server, wherein the authorization code indicates a validity of the activation code;
- activating, by the diabetes management application (14), the medical feature using the activation code, the activation being performed in response to the activation code being valid, the medical feature being a piece of program logic causing the mobile device to display medical data;
- configuring, by the diabetes management application, the activated medical feature using at least one of the one or more parameters associated with the prescription;
- generating, using the activated medical feature, data based on the one or more parameters associated with the prescription, wherein the generated data is indicative of how a drug shall be administered to a mammal by an apparatus for administering said drug;
- sending the generated data to the apparatus for administering the drug;
- pairing, by an interface of the glucose-meter, the glucose-meter with the diabetes management application (14), wherein in a paired state the glucose-meter and the diabetes management application (14) are connected via a data communication connection and whereby the pairing is such that only in the paired state the one medical feature of the diabetes management application, if successfully activated, is configured to receive measurement data from said glucose-meter and use said measurement data as input for the generating of the data being indicative of how the drug shall be administered, the measurement data comprising a glucose level of the mammal;
- assigning, by the glucose-meter, a unique sequence number to each blood glucose measure and tagging each glucose measure with a time stamp and a serial number for the glucose meter;
- transmitting, by the glucose-meter, each blood glucose measure individually to the mobile device;
- storing, by the glucose-meter, the blood glucose measures in a memory of the glucose meter; and
- displaying, by the glucose-meter, the glucose measures to the user on a display of the glucose meter;
- determining, by the diabetes management application, upon receiving a glucose measure, a sequence number associated with the received glucose measure;
- comparing, by the diabetes management application, the determined sequence number with the sequence numbers of a series of glucose measures previously received from the glucose meter;
- transmitting a request for missing glucose measures to the glucose meter when an omission in the sequence is detected; and
- receiving, by the glucose-meter, a request for missing glucose measures from the diabetes management application, the request identifying any missing glucose measures by its sequence number; and
- transmitting, by the glucose-meter in response to receiving said request, the missing glucose measures to the diabetes management application.

8. The method of claim 7, wherein the generated data comprises one or more of:
- bolus dosage data, the bolus dosage data being indicative of the amount of the drug to be administered;
- a treatment scheme for administering the drug;
- an indication of one or more time moments for administering the drug;
- a warning message not to administer the drug;
- a human-readable advice how to use the apparatus for administering the drug.

9. The method of any one of claims 7-8,
- wherein the generated data sent to the apparatus for administering the drug comprises configuration data, the method comprising configuring the apparatus for administering the drug such that it is configured to administer the drug in an amount, in a composition and/or at a time as specified in said configuration data; and/or
- wherein the generated data sent to the apparatus for administering the drug comprises command data, wherein the method further comprises triggering, by the sent command data, the apparatus for administering the drug to immediately administer the drug upon receipt of the command data, whereby the drug is administered in an amount as specified in said command data.

10. The method of any one of claims 7-9, further comprising
- the diabetes management application initiating a pairing between the apparatus for administering the drug and the diabetes management application (14), wherein in a paired state the apparatus for administering the drug and the diabetes management application (14) are connected via a data communication connection; whereby the pairing is such that only in the paired state the one medical feature of the diabetes management application, if successfully activated, is configured to send the generated data being indicative of how the drug shall be administered to the apparatus for administering the drug.

11. The method of any one of claims 7-10, further comprising:
- the diabetes management application initiating a pairing between the a measurement device and the diabetes management application (14), wherein in a paired state the measurement device and the diabetes management application (14) are connected via a data communication connection; and whereby the pairing is such that only in the paired state the one medical feature of the diabetes management application, if successfully activated, is configured to receive measurement data from said measurement device and use said measurement data as input for the generating of the data being indicative of how the drug shall be administered.

12. The method of any one of claims 7-11, wherein the authorization code indicates that the activation code is valid in response to confirming that:
- the activation code is received from the diabetes management application and not from any other diabetes management application;
- the activation code is for use in a geographic area where the diabetes management application is being used, wherein the geographic area is, for example, a country;
- the activation code has not been used before; and
- the activation code has not been disabled as a result of being lost or stolen.

13. The method of any one of claims 7-12, wherein the method is implemented in a treatment apparatus according to any one of the claims 1-6.

14. The method of any one of claims 7-13, wherein the authorization code includes the following:
- a country code indicating a country in which the activation code is to be used;
- the identifier that identifies the medical feature;
- the activation code;
- status indicating whether the activation code is valid; and
- a time stamp indicating date and time when the activation code is validated.

15. The method of any one of claims 7-14, wherein the activation code includes an alphanumeric string prefixed with a country code and the identifier that identifies the medical feature.

16. The method of any one of claims 7-15, wherein the activation code is valid for a predetermined time period and expires at an end of the predetermined time period.

17. The method of any one of claims 7-16, wherein the one or more parameters associated with the prescription include a set of parameters that are paired with the activation code and that are used to setup the activated medical feature of the diabetes management application.

18. The method of any one of claims 7-17, wherein:
- the medical feature is a physician prescribable feature; or
- the medical feature is a bolus advisor feature and the generated data are bolus dosage data, wherein a 'bolus advisor feature' is a piece of program logic operable to calculate and output a bolus via a user interface.

19. The method of any one of the previous claims 7-18, wherein the alphanumeric string includes four alphanumeric characters, wherein the country code includes two characters, and wherein the identifier includes one numeral.

20. The method of any one of claims 7-19, wherein the set of parameters includes:
- a target range, which includes acceptable upper and lower blood glucose levels when fasting or before a meal;
- meal rise, which accommodates an expected rise in blood glucose levels in response to food intake;
- a carb ratio, which is an amount of insulin necessary to account for a specified amount of carbohydrates;
- an insulin sensitivity, which is an amount of insulin necessary to lower blood glucose levels by a specified amount; and
- a maximum bolus, which is a maximum amount of insulin to be delivered at one time.

21. The method of any one of the claims 7-20, wherein the one or more parameters associated with the prescription include a set of parameters that are not paired with the activation code and that are used to generate bolus dosage data, and wherein the set of parameters includes one or more of the following:
- a snack size, which defines a threshold of carbohydrates above which a meal rise is triggered;
- an offset time, which is a length of time after a bolus is administered until a reduction in blood glucose levels begins;
- an acting time, which is a length of time insulin is expected to be effective at lowering blood glucose levels;
- insulin increment, which a unit or a fraction of a unit of insulin; and
- hypo, which is a setting below which blood glucose levels are considered hypoglycemic.

22. The method of any one of claims 7-21, the method comprising:
- receiving, by a healthcare professional, an activation code from a server (18) to activate the medical feature of the diabetes management application, wherein the activation code includes (i) a country code of a country where the activation code is to be used, (ii) an identifier that identifies the medical feature to be activated, and (iii) an alphanumeric string;
- wherein the diabetes management application (14) receives (i) the activation code and (ii) one or more parameters associated with a prescription from the healthcare professional.

## Patentansprüche

1. Behandlungsapparat, umfassend:
- ein Blutzuckermessgerät (12);
- einen Apparat zur Verabreichung eines Arzneistoffs an einen Säuger; und
- eine mobile Vorrichtung (16);
wobei die mobile Vorrichtung über ein Netz mit einem Server (18) verbunden ist;
wobei in der mobilen Vorrichtung eine Diabeteskontrollanwendung (14) untergebracht ist;
wobei die Diabeteskontrollanwendung ein oder mehrere medizinische Einrichtungen umfasst, wobei eine medizinische Einrichtung eine Programmlogik ist, die bewirkt, dass die mobile Vorrichtung medizinische Daten anzeigt;
wobei das Anwendungsprogramm dafür ausgelegt ist, die folgenden Schritte auszuführen:
- Empfangen (i) eines Aktivierungscodes zum Aktivieren einer oder mehrerer medizinischer Einrichtungen der Diabeteskontrollanwendung und (ii) eines oder mehrerer Parameter, die mit einer Verschreibung assoziiert sind, wobei der Aktivierungscode von einer Person mit Diabetes in die Diabeteskontrollanwendung eingegeben wird und eine Kennung aufweist, die eine der medizinischen Einrichtungen identifiziert, die aktiviert werden soll;
- durch die Diabeteskontrollanwendung: Senden des Aktivierungscodes über das Netz von der mobilen Vorrichtung an den Server (18);
- durch die Diabeteskontrollanwendung: Empfangen eines Autorisierungscodes vom Server, wobei der Autorisierungscode eine Gültigkeit des Aktivierungscodes angibt;
- durch die Diabeteskontrollanwendung (14): Aktivieren der einen angegebenen medizinischen Einrichtung unter Verwendung des Aktivierungscodes, wobei die Aktivierung als Reaktion darauf durchgeführt wird, dass der Aktivierungscode gültig ist;
- durch die Diabeteskontrollanwendung: Konfigurieren der aktivierten medizinischen Einrichtung unter Verwendung von mindesten einem von dem einen oder den mehreren Parametern, die mit der Beschreibung assoziiert sind; und
- Erzeugen von Daten auf Basis des einen oder der mehreren Parameter, die mit der Verschreibung assoziiert sind, unter Verwendung der aktivierten medizinischen Einrichtung, wobei die erzeugten Daten angeben, wie der Arzneistoff verabreicht werden soll;
- Senden der erzeugten Daten an den Apparat zur Verabreichung des Arzneistoffs;- wobei das Blutzuckermessgerät einen Prozessor und eine Schnittstelle zum Austauschen von Daten mit der Diabeteskontrollanwendung (14) umfasst, wobei die Schnittstelle dafür ausgelegt ist, das Blutzuckermessgerät mit der Diabeteskontrollanwendung zu koppeln;
- wobei das Blutzuckermessgerät und die Diabeteskontrollanwendung (14) in einem Kopplungszustand über eine Datenkommunikationsverbindung verbunden sind; und wobei die Kopplung so ist, dass die eine medizinische Einrichtung der Diabeteskontrollanwendung dafür ausgelegt ist, nur im verkoppelten Zustand, falls sie erfolgreich aktiviert worden ist, Messdaten vom Blutzuckermessgerät zu empfangen und die Messdaten als Eingabe für die Erzeugung der Daten zu verwenden, die angeben, wie der Arzneistoff verabreicht werden soll, wobei die Messdaten einen Blutzuckerspiegel des Säugers umfassen;
- wobei das Blutzuckermessgerät dafür ausgelegt ist, jedem Blutzuckermessergebnis eine eindeutige Folgenummer zuzuweisen, jedes Blutzuckermessergebnis mit einem Zeitstempel und einer Seriennummer für das Blutzuckermessgerät zu markieren, jedes Blutzuckermessergebnis einzeln an die mobile Vorrichtung zu senden, die Blutzuckermessergebnisse in einem Speicher des Blutzuckermessgeräts zu speichern und sie dem Anwender auf einer Anzeige des Blutzuckermessgeräts anzuzeigen; und
- wobei die Diabeteskontrollanwendung dafür ausgelegt ist, nach Empfang eines Blutzuckermessergebnisses eine Folgenummer, die mit dem empfangenen Blutzuckermessergebnis assoziiert ist, zu bestimmen; die bestimmte Folgenummer mit den Folgenummern einer Abfolge von Blutzuckermessergebnissen zu vergleichen, die zuvor vom Blutzuckermessgerät (12) empfangen wurden; und eine Abfrage von fehlenden Blutzuckermessergebnissen zu senden, wenn eine Lücke in der Folge erfasst wird; und
- wobei das Blutzuckermessgerät dafür ausgelegt ist, eine Abfrage von fehlenden Blutzuckermessergebnissen von der Diabeteskontrollanwendung zu empfangen, wobei die Abfrage etwaige fehlende Blutzuckermessergebnisse anhand von deren Folgenummern identifiziert, und als Reaktion auf den Empfang der Abfrage die fehlenden Blutzuckermessergebnisse an die Diabeteskontrollanwendung zu senden.

2. Behandlungsapparat nach Anspruch 1, wobei die erzeugten Daten eine oder mehrere der folgenden umfassen:
- Bolusdosisdaten, wobei die Bolusdosisdaten die Menge des Arzneistoffs angeben, die verabreicht werden soll;
- ein Behandlungsschema für die Verabreichung des Arzneistoffs;
- eine Angabe eines oder mehrerer Zeitpunkte für die Verabreichung des Arzneistoffs;
- einen Warnhinweis, wann der Arzneistoff nicht verabreicht werden darf;
- eine von einem Menschen lesbare Empfehlung, wie der Apparat zur Verabreichung des Arzneistoffs anzuwenden ist.

3. Behandlungsapparat nach einem der vorangehenden Ansprüche, wobei die erzeugten Daten, die an den Apparat zur Verabreichung des Arzneistoffs gesendet werden, umfassen:
- Konfigurationsdaten, wobei der Apparat zur Verabreichung des Arzneistoffs dafür ausgelegt ist, den Arzneistoff in einer Menge, mit einer Zusammensetzung und/oder zu einer Zeit zu verabreichen wie in den Konfigurationsdaten angegeben; und/oder
- Befehlsdaten, wobei der Apparat zur Verabreichung des Arzneistoffs dafür ausgelegt ist, den Arzneistoff unmittelbar nach dem Empfang der Befehlsdaten zu verabreichen, wobei der Arzneistoff in einer Menge verabreicht wird, die in den Befehlsdaten vorgegeben ist.

4. Behandlungsapparat nach einem der vorangehenden Ansprüche,
- wobei der Apparat zur Verabreichung des Arzneistoffs einen Prozessor und eine Schnittstelle zum Austauschen von Daten mit der Diabeteskontrollanwendung umfasst; und
- wobei die Schnittstelle dafür ausgelegt ist, den Apparat zur Verabreichung des Arzneistoffs mit der Diabeteskontrollanwendung (14) zu koppeln;
- wobei der Apparat zur Verabreichung des Arzneistoffs und die Diabeteskontrollanwendung (14) in einem Kopplungszustand über eine Datenkommunikationsverbindung verbunden sind; und
- wobei die Kopplung so ist, dass die eine medizinische Einrichtung der Diabeteskontrollanwendung dafür ausgelegt ist, nur im Kopplungszustand, falls sie erfolgreich aktiviert worden ist, die erzeugten Daten, die angeben, wie der Arzneistoff verabreicht werden soll, an den Apparat zur Verabreichung des Arzneistoffs zu senden.

5. Behandlungsapparat nach einem der vorangehenden Ansprüche, wobei der Apparat zur Verabreichung des Arzneistoffs eine Insulinpumpe ist und wobei der Arzneistoff Insulin ist.

6. Behandlungsapparat nach einem der vorangehenden Ansprüche, ferner umfassend:
- einen Server;
wobei der Server ausgelegt ist zum:
- Erzeugen des Aktivierungscodes;
- Bereitstellen des erzeugten Aktivierungscodes direkt oder indirekt an der Diabeteskontrollanwendung (14), die auf der mobilen Vorrichtung läuft;
- Speichern einer Kopie des erzeugten Aktivierungscodes als Referenzwert;
- Empfangen des Aktivierungscodes von der Diabeteskontrollanwendung über das Netz;
- Prüfen, ob der empfangene Aktivierungscode gültig ist, wobei das Prüfen ein Vergleichen des empfangenen Aktivierungscodes mit dem gespeicherten Referenzwert umfasst;
- Zurückschicken des Authentifizierungscodes an die Diabeteskontrollanwendung (14), falls der empfangene Aktivierungscode gültig ist.

7. Verfahren zum Aktivieren einer medizinischen Einrichtung einer Diabeteskontrollanwendung (14), die an einer mobilen Vorrichtung (16) und einem Blutzuckermessgerät ausgeführt wird, wobei das Verfahren umfasst:
- durch die Diabeteskontrollanwendung: Empfangen (i) eines Aktivierungscodes zum Aktivieren der medizinischen Einrichtung der Diabeteskontrollanwendung und (ii) eines oder mehrerer Parameter, die mit einer Verschreibung assoziiert sind, wobei der Aktivierungscode von einer Person mit Diabetes in die Diabeteskontrollanwendung eingegeben wird und eine Kennung aufweist, welche die medizinische Einrichtung identifiziert, die aktiviert werden soll;
- durch die Diabeteskontrollanwendung: Senden des Aktivierungscodes über ein Netz von der mobilen Vorrichtung an einen Server (18);
- durch die Diabeteskontrollanwendung: Empfangen eines Autorisierungscodes vom Server, wobei der Autorisierungscode eine Gültigkeit des Aktivierungscodes angibt;
- durch die Diabeteskontrollanwendung (14): Aktivieren der medizinischen Einrichtung unter Verwendung des Aktivierungscodes, wobei die Aktivierung als Reaktion darauf durchgeführt wird, dass der Aktivierungscode gültig ist, wobei die medizinische Einrichtung eine Programmlogik ist, die bewirkt, dass der mobile Apparat medizinische Daten anzeigt;
- durch die Diabeteskontrollanwendung: Konfigurieren der aktivierten medizinischen Einrichtung unter Verwendung von mindesten einem von dem einen oder den mehreren Parametern, die mit der Verschreibung assoziiert sind;
- Erzeugen von Daten auf Basis des einen oder der mehreren Parameter, die mit der Verschreibung assoziiert sind, unter Verwendung der aktivierten medizinischen Einrichtung, wobei die erzeugten Daten angeben, wie ein Arzneistoff einem Säuger durch einen Apparat zur Verabreichung des Arzneistoffs verabreicht werden soll;
- Senden der erzeugten Daten an den Apparat zur Verabreichung des Arzneistoffs;
- durch eine Schnittstelle des Blutzuckermessgeräts: Koppeln des Blutzuckermessgeräts und der Diabeteskontrollanwendung (14), wobei das Blutzuckermessgerät und die Diabeteskontrollanwendung (14) in einem Kopplungszustand über eine Datenkommunikationsverbindung verbunden sind; und wobei die Kopplung so ist, dass die eine medizinische Einrichtung der Diabeteskontrollanwendung dafür ausgelegt ist, nur im verkoppelten Zustand, falls sie erfolgreich aktiviert worden ist, Messdaten vom Blutzuckermessgerät zu empfangen und die Messdaten als Eingabe für die Erzeugung der Daten zu verwenden, die angeben, wie der Arzneistoff verabreicht werden soll, wobei die Messdaten einen Blutzuckerspiegel des Säugers umfassen;
- durch das Blutzuckermessgerät: Zuordnen einer eindeutigen Folgenummer zu jedem Blutzuckermessergebnis und Markieren jedes Blutzuckermessergebnisses mit einem Zeitstempel und einer Seriennummer für das Blutzuckermessgerät;
- durch das Blutzuckermessgerät: Senden jedes Blutzuckermessergebnisses einzeln an die mobile Vorrichtung;
- durch das Blutzuckermessgerät: Speichern der Blutzuckermessergebnisse in einem Speicher des Blutzuckermessgeräts; und
- durch das Blutzuckermessgerät: Anzeigen der Blutzuckermessergebnisse für den Anwender auf einer Anzeige des Blutzuckermessgeräts;
- durch die Diabeteskontrollanwendung: Bestimmen einer Folgenummer, die mit einem empfangenen Blutzuckermessergebnis assoziiert ist, sobald das Blutzuckermessergebnis empfangen wird;
- durch die Diabeteskontrollanwendung: Vergleichen der bestimmten Folgenummer mit den Folgenummern einer Abfolge von Blutzuckermessergebnissen, die zuvor vom Blutzuckermessgerät empfangen wurden;
- Senden einer Anforderung von fehlenden Blutzuckermessergebnissen an das Blutzuckermessgerät, wenn eine Lücke in der Folge erfasst wird; und
- durch das Blutzuckermessgerät: Empfangen einer Anforderung von fehlenden Blutzuckermessergebnissen von der Diabeteskontrollanwendung, wobei die Anforderung etwaige fehlende Blutzuckermessergebnisse anhand von deren Folgenummern identifiziert; und
- Senden der fehlenden Blutzuckermessergebnisse an die Diabeteskontrollanwendung durch das Blutzuckermessgerät als Reaktion auf den Empfang der Anforderung.

8. Verfahren nach Anspruch 7, wobei die erzeugten Daten eine oder mehrere der folgenden umfassen:
- Bolusdosisdaten, wobei die Bolusdosisdaten die Menge des Arzneistoffs angeben, die verabreicht werden soll;
- ein Behandlungsschema für die Verabreichung des Arzneistoffs;
- eine Angabe eines oder mehrerer Zeitpunkte für die Verabreichung des Arzneistoffs;
- einen Warnhinweis, wann der Arzneistoff nicht verabreicht werden darf;
- eine von einem Menschen lesbare Empfehlung, wie die Vorrichtung zur Verabreichung des Arzneistoffs anzuwenden ist.

9. Verfahren nach einem der Ansprüche 7-8,
- wobei die erzeugten Daten, die an den Apparat zur Verabreichung des Arzneistoffs gesendet werden, Konfigurationsdaten umfassen, wobei das Verfahren das Konfigurieren des Apparats zur Verabreichung des Arzneistoffs auf solche Weise umfasst, dass dieser dafür ausgelegt ist, den Arzneistoff in einer Menge, mit einer Zusammensetzung und/oder zu einer Zeit zu verabreichen, wie in den Konfigurationsdaten vorgegeben ist; und/oder
- wobei die erzeugten Daten, die an den Apparat zur Verabreichung des Arzneistoffs gesendet werden, Befehlsdaten umfassen, wobei das Verfahren ferner umfasst, dass der Apparat zur Verabreichung des Arzneistoffs durch die gesendeten Befehlsdaten veranlasst wird, den Arzneistoff sofort nach Empfang der Befehlsdaten zu verabreichen, wobei der Arzneistoff in einer Menge verabreicht wird, die in den Befehlsdaten vorgegeben ist.

10. Verfahren nach einem der Ansprüche 7-9, ferner umfassend:
- dass die Diabeteskontrollanwendung eine Kopplung zwischen dem Apparat zur Verabreichung des Arzneistoffs und der Diabeteskontrollanwendung (14) initiiert, wobei in einem Kopplungszustand der Apparat zur Verabreichung des Arzneistoffs und die Diabeteskontrollanwendung (14) über eine Datenkommunikationsverbindung verbunden sind; wobei die Kopplung so ist, dass die eine medizinische Einrichtung der Diabeteskontrollanwendung dafür ausgelegt ist, nur im Kopplungszustand, falls sie erfolgreich aktiviert worden ist, die erzeugten Daten, die angeben, wie der Arzneistoff verabreicht werden soll, an den Apparat zur Verabreichung des Arzneistoffs zu senden.

11. Verfahren nach einem der Ansprüche 7-10, ferner umfassend:
- dass die Diabeteskontrollanwendung eine Kopplung zwischen dem Blutzuckermessgerät und der Diabeteskontrollanwendung (14) initiiert, wobei das Blutzuckermessgerät und die Diabeteskontrollanwendung (14) in einem Kopplungszustand über eine Datenkommunikationsverbindung verbunden sind; und wobei die Kopplung so ist, dass die eine medizinische Einrichtung der Diabeteskontrollanwendung dafür ausgelegt ist, nur im verkoppelten Zustand, falls sie erfolgreich aktiviert worden ist, Messdaten vom Blutzuckermessgerät zu empfangen und die Messdaten als Eingabe für die Erzeugung der Daten zu verwenden, die angeben, wie der Arzneistoff verabreicht werden soll.

12. Verfahren nach einem der Ansprüche 7-11, wobei der Autorisierungscode angibt, dass der Aktivierungscode gültig ist, als Reaktion auf eine Bestätigung, dass:
- der Aktivierungscode von der Diabeteskontrollanwendung und nicht von irgendeiner anderen Diabeteskontrollanwendung empfangen worden ist;
- der Aktivierungscode zur Verwendung in einem geografischen Gebiet bestimmt ist, wo die Diabeteskontrollanwendung verwendet wird, wobei das geografische Gebiet beispielsweise ein Land ist;
- der Aktivierungscode zuvor nicht verwendet wurde; und
- der Aktivierungscode nicht als Ergebnis eines Verlustes oder Diebstahls außer Kraft gesetzt worden ist.

13. Verfahren nach einem der Ansprüche 7-12, wobei das Verfahren in einem Behandlungsapparat nach einem der Ansprüche 1-6 implementiert wird.

14. Verfahren nach einem der Ansprüche 7-13, wobei der Autorisierungscode Folgendes einschließt:
- einen Ländercode, der ein Land angibt, in dem der Aktivierungscode verwendet werden soll;
- die Kennung, welche das medizinische Einrichtung identifiziert;
- den Aktivierungscode;
- einen Status, der angibt, ob der Aktivierungscode gültig ist; und
- einen Zeitstempel, der ein Datum und eine Zeit angibt, wann die Gültigkeit des Aktivierungscodes festgestellt wurde.

15. Verfahren nach einem der Ansprüche 7-14, wobei der Aktivierungscode eine alphanumerische Zeichenfolge umfasst, der ein Ländercode und die Kennung, welche die medizinische Einrichtung identifiziert, vorangestellt sind.

16. Verfahren nach einem der Ansprüche 7-15, wobei der Aktivierungscode für eine vorgegebene Zeitdauer gültig ist und am Ende der vorgegebenen Zeitdauer verfällt.

17. Verfahren nach einem der Ansprüche 7-16, wobei der eine oder die mehreren Parameter, die mit der Verschreibung assoziiert sind, einen Satz von Parametern einschließen, die mit dem Aktivierungscode gekoppelt sind und die verwendet werden, um die aktivierte medizinische Einrichtung der Diabeteskontrollanwendung einzurichten.

18. Verfahren nach einem der Ansprüche 7-17, wobei:
- die medizinische Einrichtung eine von einem Arzt verschreibbare Einrichtung ist; oder
- die medizinische Einrichtung eine Bolusempfehlungseinrichtung ist und die erzeugten Daten Bolusdosisdaten sind, wobei eine 'Bolusempfehlungseinrichtung' eine Programmlogik ist, die dazu dient, einen Bolus über eine Benutzeroberfläche zu berechnen und auszugeben.

19. Verfahren nach einem der vorangehenden Ansprüche 7-18, wobei die alphanumerische Zeichenfolge vier alphanumerische Buchstaben einschließt, wobei der Ländercode zwei Buchstaben einschließt und wobei die Kennung eine Ziffer einschließt.

20. Verfahren nach einem der Ansprüche 7-19, wobei der Satz von Parametern einschließt:
- einen Zielbereich, der annehmbare obere und untere Blutzuckerspiegel für einen nüchternen Zustand oder vor einer Mahlzeit einschließt;
- einen postprandialen Anstieg, der einen erwarteten Anstieg von Blutzuckerspiegeln als Reaktion auf eine Nahrungsaufnahme berücksichtigt;
- ein Kohlehydratverhältnis, wobei es sich um eine Menge an Insulin handelt, die nötig ist, um eine bestimmte Menge an Kohlehydraten zu bewältigen;
- eine Insulinempfindlichkeit, wobei es sich um eine Menge an Insulin handelt, die nötig ist, um Blutzuckerspiegel in einem bestimmten Maß zu senken; und
- einen maximalen Bolus, wobei es sich um eine maximale Menge an Insulin handelt, die auf einmal abgegeben werden kann.

21. Verfahren nach einem der Ansprüche 7-20, wobei der eine oder die mehreren Parameter, die mit der Verschreibung assoziiert sind, einen Satz von Parametern einschließen, die nicht mit dem Aktivierungscode gekoppelt sind und die verwendet werden, um Bolusdosisdaten zu erzeugen, und wobei der Satz von Parametern einen oder mehrere der folgenden einschließt:
- die Größe eines Imbisses, die einen Grenzwert für Kohlehydrate angibt, jenseits von dem ein postprandialer Anstieg ausgelöst wird;
- eine Verzögerung, bei der es sich um eine Länge einer Zeit nach der Verabreichung eines Bolus handelt, bis eine Senkung von Blutzuckerspiegeln einsetzt;
- eine Wirkungszeit, wobei es sich um eine Länge der Zeit handelt, über die erwartet wird, dass Insulin wirksam ist, um Blutzuckerspiegel zu senken;
- ein Insulininkrement, wobei es sich um eine Einheit oder Fraktion einer Einheit von Insulin handelt; und
- Hypo, wobei es sich um eine Einstellung handelt, unterhalb derer Blutzuckerspiegel als hypoglykämisch betrachtet werden.

22. Verfahren nach einem der Ansprüche 7-21, wobei das Verfahren ferner umfasst:
- Empfangen eines Aktivierungscodes durch eine medizinisch ausgebildete Person von einem Server (18), um die medizinische Einrichtung der Diabeteskontrollanwendung zu aktivieren, wobei der Aktivierungscode umfasst: (i) einen Ländercode eines Landes, wo der Aktivierungscode verwendet werden soll, (ii) eine Kennung, welche die medizinische Einrichtung identifiziert, die aktiviert werden soll, und (iii) eine alphanumerische Zeichenfolge;
- wobei die Diabeteskontrollanwendung (14) (i) den Aktivierungscode und (ii) einen oder mehrere Parameter empfängt, die mit einer Verschreibung durch die medizinisch ausgebildete Person assoziiert sind.

## Revendications

1. Appareil de traitement comprenant :
- un glucose-mètre (12) ;
- un appareil d'administration d'un médicament à un mammifère ; et
- un dispositif mobile (16) ;
où le dispositif mobile est connecté à un serveur (18) par le biais d'un réseau ;
où le dispositif mobile est l'hôte d'une application de gestion du diabète (14) ;
où l'application de gestion du diabète comprend une ou plusieurs fonctionnalités médicales, où une fonctionnalité médicale est un élément d'une logique de programme faisant en sorte que le dispositif mobile affiche des données médicales ;
où le programme d'application est configuré pour exécuter les étapes suivantes :
- de réception (i) d'un code d'activation pour activer une des fonctionnalités médicales de l'application de gestion du diabète et (ii) un ou plusieurs paramètres associés avec une prescription, où le code d'activation est entré par une personne ayant du diabète dans l'application de gestion du diabète et inclut un identifiant qui identifie une des fonctionnalités médicales qui doit être activée ;
- de transmission, par l'application de gestion du diabète, du code d'activation par le biais du réseau du dispositif mobile vers le serveur (18) ;
- de réception, par l'application de gestion du diabète, d'un code d'autorisation provenant du serveur, où le code d'autorisation indique une validité du code d'activation ;
- d'activation, par l'application de gestion du diabète (14), de la fonctionnalité médicale en question indiquée en utilisant le code d'activation, l'activation étant effectuée en réponse au code d'activation en cours de validité ;
- de configuration, par l'application de gestion du diabète, de la fonctionnalité médicale activée en utilisant au moins un ou plusieurs paramètres associés avec la prescription ; et
- de création, en utilisant la fonctionnalité médicale activée, de données basées sur le ou les paramètres associés avec la prescription, les données créées étant indicatrices de la manière dont le médicament devrait être administré ;
- d'envoi des données créées à l'appareil d'administration du médicament ;
- où le glucose-mètre comprend un processeur et une interface pour échanger des données avec l'application de gestion du diabète (14), l'interface étant configurée pour apparier le glucose-mètre à l'application de gestion du diabète ;
- où, dans un état apparié, le glucose-mètre et l'application de gestion du diabète (14) sont connectés par le biais d'une connexion de communication de données ; et l'appariement est ainsi fait qu'uniquement dans l'état apparié, la fonctionnalité médicale en question de l'application de gestion du diabète, si elle est activée avec succès, est configurée pour recevoir des données de mesure dudit glucose-mètre et pour utiliser les dites données de mesure comme des entrées pour la création des données étant indicatrices de la manière dont le médicament doit être administré, les données de mesure comprenant un taux de glucose d'un mammifère ;
- où le glucose-mètre est configuré pour attribuer un numéro de séquence unique à chaque mesure de glucose sanguin, pour repérer chaque mesure de glucose avec un horodatage et un numéro de série pour le glucose-mètre, pour transmettre chaque mesure de glucose sanguin individuellement au dispositif mobile, pour stocker les mesures de glucose sanguin dans une mémoire du glucose-mètre et pour être affichées pour l'utilisateur sur un affichage du glucose-mètre ; et
- où l'application de gestion du diabète est configurée pour déterminer, lors de la réception d'une mesure de glucose, un numéro de séquence associé avec la mesure de glucose reçu ; pour comparer le numéro de séquence déterminé avec les numéros de séquence d'une série de mesures de glucose précédemment reçues du glucose-mètre (12) ; et pour transmettre une demande pour des mesures de glucose manquantes lorsqu'une omission dans la séquence est détectée ; et
- où le glucose-mètre est configuré pour recevoir une demande pour des mesures de glucose manquantes à partir de l'application de gestion du diabète, la demande identifiant toute mesure de glucose manquante par son numéro de séquence, et pour transmettre, en réponse à ladite demande de réception, les mesures de glucose manquantes à l'application de gestion du diabète.

2. Appareil de traitement selon la revendication 1, dans lequel les données générées comprennent un ou plusieurs éléments parmi :
- une donnée de dosage de bolus, la donnée de dosage de bolus étant indicatrice de la quantité de médicament à administrer ;
- un schéma de traitement pour l'administration du médicament ;
- une indication d'un ou de plusieurs moments pour l'administration du médicament ;
- un message d'avertissement de ne pas administrer le médicament ;
- un conseil lisible par un humain sur la manière d'utiliser l'appareil pour administrer le médicament.

3. Appareil de traitement selon l'une quelconque des revendications précédentes, dans lequel les données générées envoyées à l'appareil pour l'administration du médicament comprennent :
- des données de configuration, où l'appareil d'administration du médicament est configuré de sorte qu'il administre le médicament dans une quantité, dans une composition et/ou à un moment tels que spécifiés dans ladite configuration ; et/ou
- des données de commande, où l'appareil d'administration du médicament est configuré de sorte qu'il administre le médicament immédiatement à la réception de la donnée de commande, où le médicament est administré dans une quantité telle que spécifiée dans la donnée de commande.

4. Appareil de traitement selon l'une quelconque des revendications précédentes,
- où l'appareil d'administration du médicament comprend un processeur et une interface pour échanger des données avec l'application de gestion du diabète ; et
- où l'interface est configurée pour apparier l'appareil d'administration du médicament avec l'application de gestion du diabète (14) ;
- où, dans un état apparié, l'appareil d'administration du médicament et l'application de gestion du diabète (14) sont connectés par le biais d'une connexion de communication de données ; et
- ce par quoi, l'appariement est tel qu'uniquement dans l'état apparié, la fonctionnalité médicale de l'application de gestion du diabète, si elle est activée avec succès, est configurée pour envoyer les données générées étant indicatrices de la manière dont le médicament devrait être administré à l'appareil d'administration du médicament.

5. Appareil de traitement selon l'une quelconque des revendications précédentes, où l'appareil d'administration du médicament est une pompe à insuline et où le médicament est de l'insuline.

6. Appareil de traitement selon l'une quelconque des revendications précédentes, comprenant en outre :
- un serveur ;
où le serveur est configuré pour :
- générer le code d'activation ;
- fournir le code d'activation directement ou indirectement à l'application de gestion du diabète (14) fonctionnant sur le dispositif mobile ;
- stocker une copie du code d'activation généré en tant que valeur de référence ;
- recevoir le code d'activation à partir de l'application de gestion du diabète par le biais du réseau ;
- vérifier si le code d'activation reçu est valide, la vérification comprenant une comparaison du code d'activation reçu avec la valeur de référence stockée ;
- dans le cas où le code d'activation est valide, le retour du code d'authentification à l'application de gestion du diabète (14).

7. Procédé d'activation d'une fonctionnalité médicale d'une application de gestion du diabète (14) exécutée sur un dispositif mobile (16) et un glucose-mètre, le procédé comprenant :
- la réception, par l'application de gestion du diabète, (i) d'un code d'activation pour activer le fonctionnalité médicale de l'application de gestion du diabète, (ii) d'un ou de plusieurs paramètres associés avec une prescription, où le code d'activation est entré par une personne diabétique dans l'application de gestion du diabète et inclut un identifiant qui identifie la fonctionnalité médicale devant être activée ;
- la transmission, par l'application de gestion du diabète, du code d'activation par le biais du réseau à partir du dispositif mobile vers un serveur (18) ;
- la réception, par l'application de gestion du diabète, d'un code d'autorisation à partir du serveur, où le code d'autorisation indique une validité du code d'activation ;
- l'activation, par l'application de gestion du diabète (14), de la fonctionnalité médicale en utilisant le code d'activation, l'activation étant effectuée en réponse à un code d'activation étant valide, la fonctionnalité médicale étant un élément d'une logique de programme faisant en sorte que le dispositif mobile affiche les données médicales ;
- la configuration, par l'application de gestion du diabète, de la fonctionnalité médicale activée en utilisant au moins un ou plusieurs paramètres associés avec la prescription ;
- la génération, en utilisant la fonctionnalité médicale activée de données basées sur le ou les paramètres associés avec la prescription, où les données générées sont indicatrices de la manière dont devrait être administré un médicament chez un mammifère par un appareil d'administration dudit médicament ;
- l'envoi des données générées à l'appareil pour l'administration du médicament ;
- l'appariement, par une interface du glucose-mètre, du glucose-mètre avec l'application de gestion du diabète (14), où dans un état apparié, le glucose-mètre et l'application de gestion du diabète (14) sont connectés par le biais d'une connexion de communication de données et ce par quoi l'appariement est tel qu'uniquement dans un état apparié, la fonctionnalité médicale donnée de l'application de gestion du diabète, si elle est activée avec succès, est configurée pour recevoir des données de mesure provenant dudit glucose-mètre et pour utiliser lesdites données de mesure en tant qu'entrée pour la génération de données étant indicatrices de la manière dont le médicament devrait être administré, les données de mesure comprenant un taux de glucose du mammifère ;
- l'attribution, par le glucose-mètre, d'un numéro de séquence unique à chaque mesure de glucose sanguin et le repérage de chaque mesure de glucose avec un horodatage et un numéro de série pour le glucose-mètre ;
- la transmission de manière individuelle par le glucose-mètre de chaque mesure de glucose sanguin vers le dispositif mobile ;
- le stockage, par le glucose-mètre, des mesures de glucose sanguin dans une mémoire du glucose-mètre ; et
- l'affichage, par le glucose-mètre, des mesures de glucose à l'utilisateur sur un affichage du glucose-mètre ;
- la détermination, par l'application de gestion du diabète lors de la réception d'une mesure de glucose, d'un numéro de séquence associé avec la mesure de glucose reçue ;
- la comparaison, par l'application de gestion du diabète, du numéro de séquence déterminé avec les numéros de séquence d'une série de mesures de glucose précédemment reçues du glucose-mètre ;
- la transmission d'une demande pour des mesures de glucose manquantes au glucose-mètre lorsqu'une omission dans la séquence est détectée ; et
- la réception, par le glucose-mètre, d'une demande pour des mesures de glucose manquantes à partir de l'application de gestion du diabète, la demande identifiant une quelconque mesure de glucose manquante par son numéro de séquence ; et
- la transmission, par le glucose-mètre en réponse à la réception de ladite demande, des mesures de glucose manquantes vers l'application de gestion du diabète.

8. Procédé selon la revendication 7, dans lequel les données générées comprennent un ou plusieurs des éléments suivants :
- une donnée de dosage de bolus, la donnée de dosage de bolus étant indicatrice de la quantité de médicament à administrer ;
- un schéma de traitement pour l'administration du médicament ;
- une indication d'un ou de plusieurs moments pour l'administration du médicament ;
- un message d'avertissement de ne pas administrer le médicament ;
- un conseil lisible par un humain sur la manière d'utiliser l'appareil pour administrer le médicament.

9. Procédé selon l'une quelconque des revendications 7 ou 8,
- dans lequel les données générées envoyées à l'appareil pour l'administration du médicament comprennent des données de configuration, le procédé comprenant une configuration de l'appareil pour l'administration du médicament de sorte qu'il est configuré pour administrer le médicament dans une quantité, dans une composition et/ou à un moment tels que spécifiés dans lesdites données de configuration ; et/ou
- dans lequel les données générées envoyées à l'appareil pour l'administration du médicament comprennent des données de commande, où le procédé comprend en outre un déclenchement, par les données de commandes envoyées, de l'appareil pour qu'il administre le médicament immédiatement à la réception de la donnée de commande, ce par quoi le médicament est administré dans une quantité telle que spécifiée dans la donnée de commande.

10. Procédé selon l'une quelconque des revendications 7 à 9, comprenant en outre
- l'initiation par l'application de gestion du diabète d'un appariement entre l'appareil d'administration du médicament et l'application de gestion du diabète (14), où, dans un état apparié, l'appareil d'administration du médicament et l'application de gestion du diabète (14) sont connectés par le biais d'une connexion de communication de données ; ce par quoi, l'appariement est tel qu'uniquement dans l'état apparié, la fonctionnalité médicale de l'application de gestion du diabète, si elle est activée avec succès, est configurée pour envoyer les données générées étant indicatrices de la manière dont le médicament devrait être administré à l'appareil d'administration du médicament.

11. Procédé selon l'une quelconque des revendications 7 à 10, comprenant en outre :
- l'initiation, par l'application de gestion du diabète, d'un appariement entre un dispositif de mesure et l'application de gestion du diabète (14), où, dans un état apparié, le dispositif de mesure et l'application de gestion du diabète (14) sont connectés par le biais d'une connexion de communication de données ; ce par quoi, l'appariement est tel qu'uniquement dans l'état apparié, la fonctionnalité médicale de l'application de gestion du diabète, si elle est activée avec succès, est configurée pour recevoir des données de mesure dudit dispositif de mesure et utiliser lesdites données de mesure en tant qu'entrée pour la génération des données étant indicatrices de la manière dont le médicament devrait être administré.

12. Procédé selon l'une quelconque des revendications 7 à 11, dans lequel le code d'autorisation indique que le code d'activation est valide en réponse à la confirmation que :
- le code d'activation est reçu à partir de l'application de gestion du diabète et non à partir d'une quelconque autre application de gestion du diabète ;
- le code d'activation est prévu pour une utilisation dans une zone géographique où l'application de gestion du diabète est en cours d'utilisation, où la zone géographique est, par exemple, un pays ;
- le code d'activation n'a pas été utilisé auparavant ; et
- le code d'activation n'a pas été désactivé en tant que conséquence d'une perte ou d'un vol.

13. Procédé selon l'une quelconque des revendications 7 à 12, où le procédé est mis en oeuvre dans un appareil de traitement selon l'une quelconque des revendications 1 à 6.

14. Procédé selon l'une quelconque des revendications 7 à 13, dans lequel le code d'autorisation inclut ce qui suit :
- un code de pays indiquant un pays dans lequel le code d'activation doit être utilisé ;
- l'identifiant qui identifie la fonctionnalité médicale ;
- le code d'activation ;
- un état indiquant si le code d'activation est valide ; et
- un horodatage indiquant la date et l'heure où le code d'activation a été validé.

15. Procédé selon l'une quelconque des revendications 7 à 14, dans lequel le code d'activation inclut une chaîne alphanumérique fixée préalablement avec un code de pays et l'identifiant qui identifie la fonctionnalité médicale.

16. Procédé selon l'une quelconque des revendications 7 à 15, dans lequel le code d'activation est valide pour une durée prédéterminée et expire à la fin de la durée prédéterminée.

17. Procédé selon l'une quelconque des revendications 7 à 16, dans lequel un ou plusieurs paramètres associés avec la prescription incluent un ensemble de paramètres qui sont appariés avec le code d'activation et qui sont utilisés pour régler la fonctionnalité médicale activée de l'application de gestion du diabète.

18. Procédé selon l'une quelconque des revendications 7 à 17, dans lequel :
- la fonctionnalité médicale est une fonctionnalité pouvant être prescrite par un médecin ; ou
- la fonctionnalité médicale est une fonctionnalité de conseil d'un bolus et les données générées sont des données de dosage de bolus, où « une fonctionnalité de conseil de bolus » est un élément d'une logique de programme pouvant fonctionner pour calculer et donner une valeur de bolus par le biais d'une interface d'utilisateur.

19. Procédé selon l'une quelconque des revendications 7 à 18, dans lequel la chaîne alphanumérique inclut quatre caractères alphanumériques, où le code de pays inclut deux caractères, et où l'identifiant inclut un chiffre.

20. Procédé selon l'une quelconque des revendications 7 à 19, dans lequel l'ensemble de paramètres inclut :
- une plage cible qui inclut des taux supérieur et inférieur de glucose sanguin acceptables au cours d'une diète ou avant un repas ;
- une augmentation due au repas qui tient compte d'une augmentation attendue des taux de glucose sanguin en réponse à l'absorption d'aliments ;
- un ratio carb qui est une quantité d'insuline nécessaire pour tenir compte d'une quantité spécifiée de carbohydrates ;
- une sensibilité à l'insuline qui est une quantité d'insuline nécessaire pour diminuer les taux de glucose sanguin d'une quantité spécifiée ; et
- un bolus maximum qui est une quantité maximale d'insuline devant être administrée en une fois.

21. Procédé selon l'une quelconque des revendications 7 à 20, dans lequel le ou les paramètres associés avec la prescription comprennent un ensemble de paramètres qui ne sont pas appariés avec le code d'activation et qui sont utilisés pour générer des données de dosage de bolus et où l'ensemble de paramètres inclut un ou plusieurs éléments suivants :
- une taille d'en-cas qui définit un seuil de carbohydrates au-dessus duquel une augmentation due au repas est déclenchée ;
- une durée d'arrêt, qui est un intervalle de temps après qu'un bolus ait été administré jusqu'à ce qu'une réduction dans les taux de glucose sanguin commence ;
- une durée d'action qui est un intervalle de temps où l'insuline est prévue être active pour la diminution des taux de glucose sanguin ;
- un incrément d'insuline qui est une unité ou une fraction d'une unité d'insuline ; et
- un hypo qui est un réglage en dessous duquel les taux de glucose sanguin sont considérés comme hypoglycémiques.

22. Procédé selon l'une quelconque des revendications 7 à 21, le procédé comprenant :
- la réception, par un professionnel de la santé, d'un code d'activation à partir d'un serveur (18), pour activer la fonctionnalité médicale de l'application de gestion du diabète, où le code d'activation inclut (i) un code de pays d'un pays où le code d'activation doit être utilisé, (ii) un identifiant qui identifie la fonctionnalité médicale devant être activée, et (iii) une chaîne alphanumérique ;
- où l'application de gestion du diabète (14) reçoit (i) le code d'activation et (ii) un ou plusieurs paramètres associés avec la prescription provenant du professionnel de santé.
